(19) Europäisches Patentamt
European Patent Office
Office européen des brevets

(11) Veröffentlichungsnummer: **0 026 369**
**B1**

(12) # EUROPÄISCHE PATENTSCHRIFT

(45) Veröffentlichungstag der Patentschrift: **08.08.84**

(21) Anmeldenummer: **80105430.5**

(22) Anmeldetag: **11.09.80**

(51) Int. Cl.³: **C 07 C 125/063**, A 61 K 31/325, C 07 D 309/14, C 07 D 309/04, C 07 D 307/22, C 07 D 307/52, C 07 D 317/28, C 07 D 319/06, C 07 D 333/20 // C07C49/573, C07C49/337, C07C49/543, C07C49/553, C07C69/96, C07C45/75, C07C45/79, C07C45/81, C07C45/62, C07C45/69, C07C68/06, C07C68/02

(54) **Neue Carbamate, ihre Herstellung und diese enthaltende pharmazeutische Zubereitungen.**

(30) Priorität: **29.09.79 DE 2939660**

(43) Veröffentlichungstag der Anmeldung: **08.04.81 Patentblatt 81/14**

(45) Bekanntmachung des Hinweises auf die Patenterteilung: **08.08.84 Patentblatt 84/32**

(84) Benannte Vertragsstaaten: **AT BE CH DE FR GB IT LI LU NL SE**

(56) Entgegenhaltungen: **DE - B - 1 122 937**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Wiersdorff, Walter-Wielant, Dr., Blockfeldstrasse 15, D-6704 Mutterstadt (DE)**
Erfinder: **Geiss, Karl-Heinz, Dr., Kirchenstrasse 8, D-6711 Beindersheim (DE)**
Erfinder: **Weifenbach, Harald, Dr., Londoner Ring 71, D-6700 Ludwigshafen (DE)**
Erfinder: **Worstmann, Wolfgang, Dr., Am Bildstock 11, D-6718 Gruenstadt (DE)**
Erfinder: **Lenke, Dieter, Dr., Kekuleplatz 1, D-6700 Ludwigshafen (DE)**
Erfinder: **Kretzschmar, Rolf, Dr., Oberer Bergelweg 4, D-6718 Gruenstadt (DE)**

EP 0 026 369 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die vorliegende Erfindung betrifft neue, von substituierten cyclischen $\beta$-Ketoalkoholen abgeleitete Carbamate, ihre Herstellung und diese Verbindungen enthaltende pharmazeutische Zubereitungen, die als Hypnotika bei der Behandlung von Schlafstörungen und als Sedativa verwendet werden können.

Die bisher bekannten Hypnotika weisen mehr oder weniger ausgeprägte Nebenwirkungen auf (D. Ginestet et al., Therapie 31, 77–103 (1976)). Aufgabe der vorliegenden Erfindung ist es, neue Hypnotika zu entwickeln.

Weiterhin ist beispielsweise aus der DE-OS 23 46 305 bekannt, daß von tricyclischen $\gamma$-Hydroxylactonen abgeleitete Carbamate, z. B. das 4-Oxa-5-(N-methylcarbamoyloxy)-tricyclo[5,2,1,$0^{2,6}$]-dec-8-en-3-on, analgetische Eigenschaften aufweisen.

Es wurde gefunden, daß Verbindungen der allgemeinen Formel I

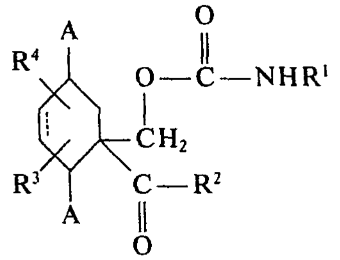

(I)

in denen die gestrichelte Linie eine Doppelbindung, die gegebenenfalls hydriert ist, bedeutet und die Reste A für Wasserstoffatome stehen oder beide Reste A zusammen eine Brücke der Formel

$$-(CH_2)_m-,$$

in der m für die Zahlen 1 oder 2 steht, bilden und $R^1$ einen gesättigten geradkettigen oder verzweigten Alkylrest mit 1 bis 6 Kohlenstoffatomen, der gegebenenfalls durch ein Halogenatom oder einen Niedrigalkoxyrest mit 1 bis 3 Kohlenstoffatomen, eine Cyanogruppe oder einen Cycloalkylrest mit 3 oder 4 Kohlenstoffatomen im Ring substituiert sein kann, einen Alkenyl- oder Alkinylrest mit 2 bis 6 Kohlenstoffatomen oder einen Cycloalkylrest mit 3 bis 6 Kohlenstoffatomen im Ring, $R^2$ Methyl oder Ethyl, $R^3$ und $R^4$ Wasserstoffatome oder Methylgruppen bedeuten, wertvolle pharmakologische Eigenschaften besitzen.

Die Begriffe Niedrigalkyl und Niedrigalkoxy sollen sich im folgenden stets auf Alkylreste mit 1 bis 3 Kohlenstoffatomen beziehen.

Es wird darauf hingewiesen, daß die Verbindungen der allgemeinen Formel I, in denen die beiden Reste A zusammen eine Brücke bilden, als exo- und endo-Verbindungen vorliegen können, wobei sich die Bezeichnung exo und endo auf die relative Stellung der Gruppe

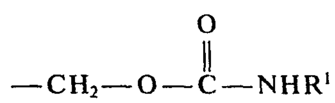

am durch die Gruppen A,A überbrückten Sechsring bezieht. Die Zuordnung erfolgt durch NMR-spektroskopischen Vergleich der

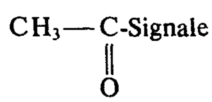
-Signale

mit exo- und endo-Norbornenylmethylketon oder durch Röntgenstrukturanalyse.

Für $R^1$ kommen als geradkettige oder verzweigte Alkylreste, als Alkenyl- oder Alkinylreste beispielsweise Methyl, Äthyl, n- oder i-Propyl, n-, i-, sec, oder tert-Butyl, n-Pentyl, 3-Pentyl, 3-Methyl-2-butyl, n-Hexyl, Äthenyl, Prop-1-enyl, Prop-1-en-2-yl, 2-Methylprop-1-en-1-yl, Hex-1-enyl, Allyl, Methallyl, But-3-en-1-yl, But-3-en-2-yl, Hex-5-en-2-yl, Prop-2-inyl, But-2-inyl, But-1-in-3-yl, 2-Methylbut-3-in-2-yl in Betracht.

Als substituierte Alkylreste für $R^1$ sind beispielsweise zu nennen: 2-Fluoräthyl, 2-Chloräthyl, 3-Chlor-1-propyl, 3-Brom-1-propyl, 1-Chlor-2-propyl, 2-Chloräthenyl, Cyanomethyl, 1-Cyanoäthyl, 2-Cyanoäthyl, 1-Cyano-2-propyl, 1-Cyano-3-hexyl, Methoxymethyl, Äthoxymethyl, Isopropoxymethyl, 1-Äthoxyäthyl, 3-Isopropoxypropyl, 1-Methoxy-2-propyl, Cyclopropylmethyl, 1-Cyclopropyläthyl, 2-Cyclopropyläthyl, 1-Cyclopropyl-2-propyl, Cyclobutylmethyl, 1-Cyclobutyläthyl, Cyclopentylmethyl, Cyclohexylmethyl.

Als Cycloalkylreste für $R^1$ können beispielsweise genannt werden: Cyclopropyl, 1-Methylcyclopropyl,

2-Methylcyclopropyl, 2,2-Dimethylcyclopropyl, 2-(2-Propyl)-cyclopropyl, Cyclobutyl, Cyclopentyl, Cyclohexyl.

Besonders bevorzugt sind Verbindungen der Formel I, in denen die gestrichelte Linie eine Doppelbindung, die gegebenenfalls hydriert sein kann, bedeutet und die Reste A für Wasserstoffatome stehen oder beide Reste A zusammen einen Methylenrest bilden, und $R^1$ einen gesättigten, geradkettigen oder verzweigten Alkylrest mit 1 bis 6 Kohlenstoffatomen, wobei ein Alkylrest mit 1 bis 3 Kohlenstoffatomen gegebenenfalls durch ein Chloratom, eine Niedrigalkoxygruppe mit 1 bis 3 Kohlenstoffatomen im Alkyl, vorzugsweise Methoxy, oder eine Cycloalkylgruppe mit 3 bis 4 Kohlenstoffatomen im Ring substituiert sein kann, oder einen Alkenyl- oder Alkinylrest mit 2 bis 6 Kohlenstoffatomen, einen Cycloalkylrest mit 3 bis 4 Kohlenstoffatomen im Ring, $R^2$ Methyl und $R^3$ und $R^4$ Wasserstoffatome bedeuten.

Neben den in den Ausführungsbeispielen angegebenen Verbindungen seien als erfindungsgemäße Verbindungen beispielsweise genannt:

2-[N-(2-Fluorpropyl)-carbamoyloxymethyl]-norborn-5-en-2-yl-methylketon
2-[N-(3-Fluorpropyl)-carbamoyloxymethyl]-norborn-5-en-2-yl-methylketon
2-[N-(1-Chlor-2-propyl)-carbamoyloxymethyl]-norborn-5-en-2-yl-methylketon
2-[N-(Prop-1-en-2-yl)-carbamoyloxymethyl]-norborn-5-en-2-yl-methylketon
2-{N-[1-(2-Methylcyclopropyl)-äthyl]-carbamoyloxymethyl}-norborn-5-en-2-yl-methylketon
2-[N-(1-Cyclobutyläthyl)-carbamoyloxymethyl]-norborn-5-en-2-yl-methylketon
2-[N-(2-Methylcyclopropyl)-carbamoyloxymethyl]-norborn-5-en-2-yl-methylketon
2-(N-But-1-en-3-ylcarbamoyloxymethyl)-norborn-5-en-2-yl-methylketon
2-(N-But-2-en-1-ylcarbamoyloxymethyl)-norborn-5-en-2-yl-methylketon
2-(N-But-3-en-1-ylcarbamoyloxymethyl)-norborn-5-en-2-yl-methylketon
2-(N-Isopropylcarbamoyloxymethyl)-3-methylnorborn-5-en-2-yl-methylketon
2-(N-Äthylcarbamoyloxymethyl)-3-methylnorborn-5-en-2-yl-methylketon
2-(N-Äthylcarbamoyloxymethyl)-5,6-dimethylnorborn-5-en-2-yl-methylketon
2-(N-Isopropylcarbamoyloxymethyl)-5,6-dimethyl-norborn-5-en-2-yl-methylketon
2-(N-Cyclopropylcarbamoyloxymethyl)-5,6-dimethyl-norborn-5-en-2-yl-methylketon
2-(N-Cyclopropylcarbamoyloxymethyl)-norborn-5-en-2-yl-äthylketon
2-[N-(1-Cyclopropyläthyl)-carbamoyloxymethyl]-norborn-5-en-2-yl-äthylketon
2-(N-Cyclopropylcarbamoyloxymethyl)-norborn-2-yl-methylketon
2-[N-(1-Cyclopropyläthyl)-carbamoyloxymethyl]-norborn-2-yl-methylketon
2-(N-Prop-1-en-2-ylcarbamoyloxymethyl)-bicyclo-[2.2.2]-oct-5-en-2-yl-methylketon
2-(N-Cyclopropylcarbamoyloxymethyl)-bicyclo-[2.2.2]-oct-5-en-2-yl-methylketon
1-(N-3-Chlorpropylcarbamoyloxymethyl)-cyclohex-3-enyl-methylketon
1-(N-2-Chloräthylcarbamoyloxymethyl)-cyclohex-3-enyl-methylketon
1-(N-Cyclopropylmethylcarbamoyloxymethyl)-cyclohex-3-enyl-methylketon
1-[N-(1-Cyclobutyläthyl)-carbamoyloxymethyl]-cyclohex-3-enyl-methylketon
1-(N-But-3-enylcarbamoyloxymethyl)-cyclohex-3-enyl-methylketon
1-(N-But-1-en-3-yl-carbamoyloxymethyl)-cyclohex-3-enyl-methylketon
1-(N-But-2-en-1-ylcarbamoyloxymethyl)-cyclohex-3-enyl-methylketon
1-(N-Allylcarbamoyloxymethyl)-cyclohex-3-enyl-methylketon
1-(N-n-Propylcarbamoyloxymethyl)-4-methylcyclohex-3-enyl-methylketon
1-(N-Isopropylcarbamoyloxymethyl)-4-methylcyclohex-3-enyl-methylketon
1-(N-Cyclopropylcarbamoyloxymethyl)-4-methylcyclohex-3-enyl-methylketon
1-[N-(1-Cyclopropyläthyl)-carbamoyloxymethyl]-4-methylcyclohex-3-enyl-methylketon
1-[N-Cyclopropylcarbamoyloxymethyl]-cyclohexyl-methylketon
1-[N-(1-Cyclopropyläthyl)-carbamoyloxymethyl]-cyclohexyl-methylketon
1-(N-Propylcarbamoyloxymethyl)-cyclohexyl-methylketon

Die erfindungsgemäßen Verbindungen der Formel I können hergestellt werden:

a) durch Umsetzung eines $\beta$-Ketoalkohols der allgemeinen Formel II,

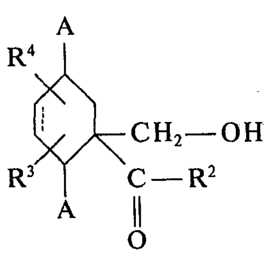

(II)

in der die gestrichelte Linie eine Doppelbindung, die gegebenenfalls hydriert sein kann, bedeutet und die Reste A und $R^2$ bis $R^4$ die für Formel I angegebenen Bedeutungen haben, mit einem Isocyanat der allgemeinen Formel III,

$R^1—N=C=O$ (III)

in der $R^1$ die für Formel I angegebenen Bedeutungen hat, oder in Form seines Säureadditionsadduktes in Gegenwart einer Base zweckmäßigerweise in einem inerten Lösungsmittel und gegebenenfalls in Anwesenheit eines Katalysators, wobei eine erhaltene ungesättigte Verbindung gegebenenfalls anschließend katalytisch hydriert wird, oder

b) durch Umsetzung eines $\beta$-Ketoalkohols der allgemeinen Formel II in Form eines Kohlensäureesters der Formel IV

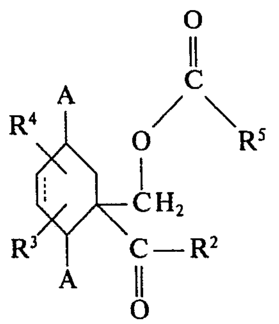

(IV)

in der A und $R^2$ bis $R^4$ die für Formel I angegebenen Bedeutungen haben und $R^5$ ein Halogenatom, vorzugsweise ein Chloratom, oder den Rest —OAr, wobei Ar eine gegebenenfalls substituierte Phenylgruppe darstellt, mit einem Amin der allgemeinen Formel V

$R^1—NH_2$ (V)

in der $R^1$ die für Formel I angegebenen Bedeutungen hat mit der Maßgabe, daß der Rest $R^1$ keine zum Stickstoffatom $\alpha,\beta$-ständige Doppel- oder Dreifachbindung aufweist, zweckmäßigerweise in einem Lösungsmittel und gegebenenfalls in Anwesenheit eines basischen Katalysators, wobei eine erhaltene ungesättigte Verbindung gegebenenfalls anschließend katalytisch hydriert wird.

Die Hydrierung einer gegebenenfalls im Ring vorhandenen Doppelbindung einer Verbindung der Formel I in an sich bekannter Weise empfiehlt sich nur, soweit unter den Hydrierungsbedingungen inerte Substituenten vorliegen.

Die Umsetzungen der Verbindungen der allgemeinen Formel II mit Isocyanaten der Formel III können ohne oder in Gegenwart eines inerten Lösungsmittels durchgeführt werden. Zweckmäßigerweise verwendet man ein organisches aprotisches Lösungsmittel, wie einen niederen gesättigten Dialkyläther, Dialkylglykoläther oder gesättigten cyclischen Äther, wie Diäthyläther, Methyl-tert-butyläther, 1,2-Dimethoxyäthan, Tetrahydrofuran oder Dioxan, einen aromatischen Kohlenwasserstoff, wie Benzol oder einen Alkylbenzol, wie Toluol oder Xylol, oder einen aliphatischen Kohlenwasserstoff, wie Hexan, Cyclohexan, Heptan oder Octan, ein niederes aliphatisches Keton, wie Aceton, Methyläthylketon oder Methylisobutylketon, einen niederen chlorierten Kohlenwasserstoff, wie Methylenchlorid, Chloroform, ein Dialkylformamid, wie Dimethyl- oder Diäthylformamid, oder ein N-Alkylaktam, wie N-Methylpyrrolidon. Die Umsetzungen können auch in Mischungen der genannten Lösungsmittel durchgeführt werden.

Die Umsetzungen werden in der Regel bei Temperaturen von 0 bis 140°C durchgeführt, bevorzugt bei Temperaturen von 20 bis 100°C.

Zweckmäßigerweise werden die Reaktionen bei Atmosphärendruck durchgeführt, sie können aber auch in einem geschlossenen Gefäß bei erhöhtem Druck erfolgen.

Die Umsetzungen der Verbindungen der allgemeinen Formel II mit Isocyanaten $R^1NCO$ kann zweckmäßigerweise in Gegenwart eines Katalysators durchgeführt werden. Als Katalysator kommen, wie dem Fachmann bekannt ist, beispielsweise eine Blei(IV)-, Zinn(II)-, Zinn(IV)-, Quecksilber(II)-Verbindung oder ein tertiäres Amin in Betracht (s. z. B. J. Appl. Polym. Sci., 13, (1969), 1929 ff; Chem. Rev. 72, 457 ff (1972)). Bevorzugte Katalysatoren sind Zinn(II)-, Zinn(IV)- oder Quecksilber(II)-Verbindungen wie z. B. Zinnoctanoat, Dibutylzinndiacetat oder Phenylquecksilberacetat.

Bevorzugte Lösungsmittel bei Umsetzungen in Gegenwart eines Katalysators, der in der Regel in Mengen von 0,1 bis 2 Gew.%, bezogen auf den eingesetzten Ketoalkohol der Formel II verwendet wird, sind niedere gesättigte Dialkyläther, Dialkylglykoläther oder cyclische Äther, wie Diäthyläther, 1,2-Dimethoxyäthan oder Tetrahydrofuran.

Für den Fall, daß $R^1$ ein Wasserstoffatom bedeutet, ist es zweckmäßig, die erforderliche Cyansäure in einem getrennten Gefäß zu erzeugen und gasförmig in die Lösung eines Ketoalkohols der Formel II einzuleiten oder aber die Cyansäure in situ zu den gewünschten Verbindungen der Formel I, $R^1 = H$, umzusetzen.

In vorteilhafter Weise wird die Cyansäure in situ aus einem Alkalimetallcyanat, beispielsweise Natrium- oder Kaliumcyanat, und einer starken Säure, wie beispielsweise Schwefelsäure oder Trifluoressigsäure, in an sich bekannter Weise (s. z. B. Loev und M. F. Kormendy, J. Org. Chem. 28 (1963) 3421) erzeugt und mit einem in der Lösung vorhandenen Ketoalkohol der Formel II umgesetzt. Auch diese Umsetzung kann ohne oder mit Lösungsmittel durchgeführt werden, geeignete Lösungsmittel sind beispielsweise Benzol oder Alkylbenzole, wie Toluol oder Xylol, niedere gesättigte Dialkyläther oder cyclische Äther, wie z. B. Diäthyläther oder Tetrahydrofuran, oder chlorierte niedere Kohlenwasserstoffe, wie Methylenchlorid. Diese Umsetzungen werden bei 0 bis 80°C, vorzugsweise 10 bis 60°C durchgeführt.

Es wird darauf hingewiesen, daß bei der Umsetzung der Ketoalkohole der allgemeinen Formel II anstelle der Isocyanate $R^1NCO$ auch ihre Säureadditionsverbindung, die Carbamidsäurechloride, $R^1$-NHCOCl verwendet werden können, wobei die Reaktionen zweckmäßigerweise in einem inerten Lösungsmittel und in Gegenwart eines säurebindenden Mittels, bevorzugt eines organischen tertiären Amins, wie Triäthylamin, N-Methylpiperidin, N-Methylpyrrolidin oder N,N-Dimethylanilin, durchgeführt werden.

Weiterhin ist es möglich, durch eine Umesterung von einem Carbaminat der Formel $R^1NHCOOR^6$, in der $R^1$ die obengenannte Bedeutung hat und $R^6$ eine Niedrigalkylgruppe oder eine Phenylgruppe, die gegebenenfalls substituiert ist, bedeutet, mit einem Ketoalkohol der allgemeinen Formel II zu einer erfindungsgemäßen Verbindung der Formel I zu gelangen.

Die Umsetzungen einer Verbindung der allgemeinen Formel IV mit einem Amin der allgemeinen Formel V können ohne oder mit Lösungsmittel durchgeführt werden, wobei das Amin $R^1NH_2$, gegebenenfalls im Überschuß eingesetzt, selbst als Lösungsmittel dienen kann. Gegebenenfalls kann das Amin in Form eines Säureadditionssalzes, insbesondere des Hydrohalogenids, in Gegenwart einer Base, z. B. der äquivalenten Menge Natriummethylat, verwendet werden.

Der Rest $R^5$ bedeutet ein Halogenatom oder einen gegebenenfalls durch 1 bis 2 elektronegative Reste, insbesondere durch Halogenatome oder die Nitrogruppe, substituierten Phenylrest, vorzugsweise steht $R^5$ für ein Chloratom oder den Phenylrest.

Die Umsetzungen werden bei Temperaturen von 0 bis 120°C durchgeführt, vorzugsweise bei Temperaturen von 20 bis 80°C.

Geeignete Lösungsmittel für diese Umsetzungen sind Wasser, niedere Alkohole mit 1 bis 4 Kohlenstoffatomen, wie Methanol, Äthanol, Isopropanol, Isobutanol, niedrige gesättigte Dialkyläther, Dialkylglykoläther oder cyclische Äther, wie Diäthyläther, Methyl-tert-butyläther, 1,2-Dimethoxyäthan, Tetrahydrofuran oder Dioxan, aromatische Kohlenwasserstoffe, wie Benzol oder ein Alkylbenzol, wie Toluol oder Xylol, aliphatische Kohlenwasserstoffe, wie Hexan, Heptan oder Cyclohexan, Dialkylformamide, wie Dimethyl- oder Diäthylformamid, chlorierte Kohlenwasserstoffe, wie Methylenchlorid oder Chloroform. Außerdem können Mischungen dieser Lösungsmittel eingesetzt werden. Bevorzugte Lösungsmittel stellen die niedrigen gesättigten Dialkyläther, Dialkylglykoläther oder cyclische Äther dar, wie Diäthyläther, Methyl-tert-butyläther, 1,2-Dimethoxymethan, Tetrahydrofuran oder Dioxan.

Die Abtrennung des bei der Reaktion entstehenden Phenols kann durch Umkristallisation der erfindungsgemäßen Verbindungen der Formel I nach dem Abdestillieren des Lösungsmittels, durch Extraktion der Reaktionslösung mit einer Alkalihydroxydlösung wie z. B. Natriumhydroxyd – gegebenenfalls nach Ersatz des Lösungsmittels in dem die Reaktion durchgeführt wurde, durch ein mit Wasser nicht vollständig mischbares Lösungsmittel – oder durch Säulenchromatographie des Rohproduktes erfolgen. Die erfindungsgemäßen Verbindungen können durch Destillation, Umkristallisation oder Säulenchromatographie weiter gereinigt werden.

Eine zweckmäßige Verfahrensweise, um eine im Ring vorhandene Doppelbindung zu hydrieren, ist es, ein entsprechendes ungesättigtes Carbamat der allgemeinen Formel I in einem unter Hydrierbedingungen inerten Lösungsmittel aufzulösen oder zu suspendieren und in Gegenwart eines Katalysators mit Wasserstoff zu hydrieren. Als Hydrierkatalysatoren sind Übergangsmetalle wie Palladium, Platin oder Nickel, gegebenenfalls auf einem Träger, wie Kohle, $CaCO_3$ oder Kieselgel, geeignet. Als Lösungsmittel kann ein niederer aliphatischer Alkohol, wie Methanol, Äthanol, Isopropanol, Isobutanol, ein niedrigerer gesättigter Dialkyläther, Dialkylglykoläther oder cyclischer Äther, wie Diäthyläther, 1,2-Dimethoxyäthan oder Tetrahydrofuran, oder ein Niedrigalkylester einer niederaliphatischen Carbonsäure, wie Essigsäuremethylester oder Essigsäureäthylester, verwendet werden. Die Umsetzung kann bei Normaldruck oder im geschlossenen Gefäß unter erhöhtem Druck bis zu 250 bar bei Raumtemperatur oder unter Wärmezufuhr, z. B. durch Erwärmen auf 30 bis 150°C, durchgeführt werden. Vorzugsweise wird die Hydrierung bei 20 bis 60°C durchgeführt.

Die Ausgangsverbindungen der allgemeinen Formel II können in an sich bekannter Weise durch Umsetzung eines Ketons der allgemeinen Formel VI

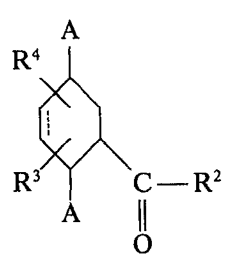

(VI)

in der $R^2$ bis $R^4$, A und B die in Formel I angegebenen Bedeutungen haben und die gestrichelte Linie eine Doppelbindung, die gegebenenfalls hydriert sein kann, bedeutet, mit Formaldehyd in Gegenwart eines basischen Katalysators erhalten werden.

Ketoalkohole der Formel II, in denen beide A eine Brücke bilden, fallen im allgemeinen als exo/endo-Gemische an, aus denen sich gegebenenfalls durch Umkristallisation oder Säulenchromatographie die reinen Isomeren gewinnen lassen.

Eine im Ring vorhandene Doppelbindung kann gegebenenfalls in an sich bekannter Weise durch katalytische Hydrierung hydriert werden.

Die Ausgangsverbindungen der Formel IV können durch Umsetzung einer Verbindung der Formel II mit einem Kohlensäurederivat der allgemeinen Formel VII

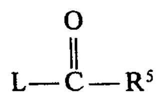

(VII)

in der $R^5$ für ein Halogenatom, vorzugsweise ein Chloratom oder die Gruppe OAr steht und L für ein Halogenatom, vorzugsweise ein Chloratom steht und falls $R^5$ den Rest OAr bedeutet, die gleiche Bedeutung wie $R^5$ besitzt und Ar eine gegebenenfalls substituierte Phenylgruppe bedeutet, erhalten werden. Zweckmäßig wird die Reaktion in Gegenwart eines basischen Katalysators, wie z. B. eines tertiären Amins, durchgeführt. In der bevorzugten Ausführungsform werden als Ausgangsmaterialien die Diarylcarbonate der Formel VII, L = OAr, verwendet.

Eine im Ring vorhandene Doppelbindung einer Verbindung der Formel IV kann gegebenenfalls auch in an sich bekannter Weise katalytisch hydriert werden.

Die erfindungsgemäßen Substanzen weisen wertvolle pharmakologische Eigenschaften auf. Sie zeichnen sich bei großer therapeutischer Breite durch eine hohe hypnotische Aktivität aus und können dementsprechend als Hypnotika bei der Behandlung von Schlafstörungen und als Sedativa verwendet werden. Im Vergleich zu den Verbindungen der DE-OS 2 346 305 zeigen sie keine analgetischen Eigenschaften.

Zur Untersuchung der hypnotischen Wirkung erhalten Gruppen von 4 bis 8 weiblichen Sprague-Dawley-Ratten im Gewicht von 145 bis 200 g die Substanzen intraperitoneal (i. p.) appliziert. Als Wirkkriterium dient die Dauer des Ausfalls des Umkehrreflexes (Schlafdauer).

Zwischen den Logarithmen der applizierten Dosen (mg/kg) und den Logarithmen der Schlafdauer (min) bestehen lineare Beziehungen, aus denen mit Hilfe der Regressionsanalyse als ED 60 min die Dosis berechnet wird, die eine Schlafdauer von 60 min bewirkt.

Die akute Toxizität wird an Gruppen von 10 Ratten desselben Stammes ebenfalls durch i. p. Applikation bestimmt.

Die LD 50 wird nach einer Beobachtungszeit von 6 Stunden mit Hilfe der Probit-Analyse berechnet.

Als Vergleichssubstanz dient das bekannte Hypnoticum Ethinamat (1-Äthinyl-cyclohexyl-carbamat).

Tabelle 1

Hypnotische Wirkung und Toxizität (Ratte, intraperitoneale Applikation)

| Verbindung Nr. | Hypnotische Wirkung ED 60 min mg/kg | R.W.[1]) | Toxizität LD 50 mg/kg | Therapeut. Breite[2]) |
|---|---|---|---|---|
| 7 | 20,7 | 3,35 | 137 | 6,6 |
| 4 | 21,5 | 3,22 | 392 | 18,2 |
| 15 | 46,4 | 1,49 | 247 | 5,3 |
| 36 | 37,6 | 1,84 | 464 | 12,3 |
| 109 | 27,2 | 2,55 | 187 | 6,9 |
| 87 | 46,7 | 1,48 | 482 | 10,3 |
| 88 | 30,8 | 2,25 | 150 | 4,9 |
| 52 | 28,5 | 2,43 | 215 | 7,5 |
| 98 | 30,3 | 2,29 | ≥681[3]) | ≥22,5 |
| Ethinamat | 69,3 | 1,00 | 296 | 4,3 |

[1]) R.W. = Relative Wirksamkeit Ethinamat = 1,00.

[2]) $\frac{\text{LD 50}}{\text{ED 60 min}}$.

[3]) Todeshäufigkeit 0/10.

Aus der Tabelle 1 geht hervor, daß die hypnotische Wirkung der erfindungsgemäßen Substanzen hoch ist. Die wirksamen Dosen für die gemäß Tabelle 1 untersuchten Verbindungen liegen an der Ratte zwischen 20,7 mg/kg (Beispiel 7) und 46,7 mg/kg (Beispiel 87). Die Wirksamkeit ist 1,5- bis 3,4mal höher als die des Ethinamats (69,3 mg/kg).

Der Abstand zwischen der letalen Dosis (LD 50) und der hypnotisch wirksamen Dosis (ED 60 min), die therapeutische Breite, liegt bei diesen Verbindungen zwischen 4,9 (Beispiel 88) und >22,5 (Beispiel 98). Sie ist größer als bei Ethinamat (4,3).

Im Hinblick auf ihre Wirkungen können als Einzelverbindungen besonders hervorgehoben werden:

exo-2-(N-Isopropylcarbamoyloxymethyl)-norborn-5-en-2-yl-methylketon
exo-2-(N-n-Propylcarbamoyloxymethyl)-norborn-5-en-2-yl-methylketon
exo-2-(N-Cyclopentylcarbamoyloxymethyl)-norborn-5-en-2-yl-methylketon
exo-2-(N-Isopropylcarbamoyloxymethyl)-norborn-2-yl-methylketon
exo-2-(N-Cyclopropylcarbamoyloxymethyl)-norborn-5-en-2-yl-methylketon
exo-2-(N-(2-Methylpropyl)-carbamoyloxymethyl)-norborn-5-en-2-yl-methylketon
exo-2-(N-But-2-ylcarbamoyloxymethyl)-norborn-5-en-2-yl-methylketon
1-(N-Propylcarbamoyloxymethyl)-cyclohex-3-en-yl-methylketon
exo-2-[N-(1-Cyclopropyläthyl)-carbamoyloxymethyl]-norborn-5-en-2-yl-methylketon

Weiterhin können beispielsweise als Verbindungen mit ähnlich guter Wirkung genannt werden:

exo-2-(N-Äthylcarbamoyloxymethyl)-norborn-5-en-2-yl-methylketon
exo-2-(N-n-Butylcarbamoyloxymethyl)-norborn-5-en-2-yl-methylketon
exo-2-(N-n-Hexylcarbamoyloxymethyl)-norborn-5-en-2-yl-methylketon
exo-2-(N-Vinylcarbamoyloxymethyl)-norborn-5-en-2-yl-methylketon
exo-2-(N-Prop-1-enylcarbamoyloxymethyl)-norborn-5-en-2-yl-methylketon
exo-2-(N-Methoxymethylcarbamoyloxymethyl)-norborn-5-en-2-yl-methylketon
endo-2-(N-Isopropylcarbamoyloxymethyl)-norborn-5-en-2-yl-methylketon

2-(N-Isopropylcarbamoyloxymethyl)-norborn-5-en-2-yl-äthylketon
exo-2-(N-Äthylcarbamoyloxymethyl)-norborn-2-yl-methylketon
exo-2-(N-n-Propylcarbamoyloxymethyl)-norborn-2-yl-methylketon
1-(N-Äthylcarbamoyloxymethyl)-cyclohex-3-en-yl-methylketon
1-(N-n-Butylcarbamoyloxymethyl)-cyclohex-3-en-yl-methylketon
1-(N-Isopropylcarbamoyloxymethyl)-cyclohex-3-en-yl-methylketon
1-(N-n-Butylcarbamoyloxymethyl)-cyclohexyl-methylketon
1-(N-Isopropylcarbamoyloxymethyl)-cyclohexyl-methylketon
exo-2-(N-n-Pentylcarbamoyloxymethyl)-norborn-5-en-2-yl-methylketon
exo-2-[N-(3-Methylbut-2-yl)-carbamoyloxymethyl]-norborn-5-en-2-yl-methylketon
exo-2-(N-Allylcarbamoyloxymethyl)-norborn-5-en-2-yl-methylketon
exo-2-(N-Propargylcarbamoyloxymethyl)-norborn-5-en-2-yl-methylketon
exo-2-[N-(Cyclopropylmethyl)-carbamoyloxymethyl]-norborn-5-en-2-yl-methylketon
exo-2-(N-Cyclobutylcarbamoyloxymethyl)-norborn-5-en-2-yl-methylketon
exo-2-[N-(2-Chloräthyl)-carbamoyloxymethyl)-norborn-5-en-2-yl-methylketon
1-(N-Cyclopropylcarbamoyloxymethyl)-cyclohex-3-en-1-yl-methylketon
1-(N-But-2-yl-carbamoyloxymethyl)-cyclohex-3-en-1-yl-methylketon
2-[N-(Cyclobutylmethyl)-carbamoyloxymethyl)-norborn-5-en-2-yl-methylketon.

Gegenstand der Erfindung sind demnach auch therapeutische Mittel oder pharmazeutische Zubereitungen, die neben den üblichen Trägerstoffen oder Verdünnungsmitteln eine Verbindung der Formel I als Wirkstoff enthalten sowie die Verwendung der erfindungsgemäßen Verbindungen als Hypnotika bei der Behandlung von Schlafstörungen und als Sedativa.

Die therapeutischen Mittel bzw. Zubereitungen werden mit den üblichen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Die Dosierung hängt von Alter, Zustand und Gewicht des Patienten sowie von der Applikationsart ab. In der Regel beträgt die Dosis 20 bis 500 mg, wobei Dosen im niedrigen Bereich für die Sedation und Dosen im höheren Bereich für die hypnotische Wirkung in Betracht kommen.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen Pulver, Granulate, Lösungen oder Suspensionen oder Depotformen.

Selbstverständlich kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Lactose, Sorbit, Mannit, Polyvinylpyrrolidon, Sprengmitteln wie Mais, Stärke oder Alginsäure, Bindemitteln wie Stärke oder Gelatine, Gleitmitteln wie Magnesiumstearat oder Talkum und/oder Mitteln zur Erzielung des Depoteffekten wie Carboxypolymethylen, Carboxymethylcellulose, Celluloseacetatphthalat oder Polyvinylacetat, erhalten werden.

Weitere übliche Zusätze sind beispielsweise Konservierungsmittel, Antioxydantien, geschmacksverbessernde Zusätze, Stabilisierungsmittel, Emulgiermittel, Netzmittel usw. Voraussetzung ist, daß alle bei der Herstellung pharmazeutischer Zubereitungen verwendeten Stoffe untoxisch und mit den verwendeten Wirkstoffen verträglich sind (vgl. L. G. Goodman, A. Gilman, The Pharmacological Basis of Therapeutics).

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Wirkstoffen können zusätzlich geschmackverbessernde Mittel wie Saccharin, Cyclamat oder Zucker sowie z. B. Aromastoffe wie Vanillin oder Orangenextrakt, enthalten. Sie können außerdem Suspendierhilfsstoffe wie Natriumcarboxymethylcellulose oder Konservierungsstoffe wie p-Hydroxybenzoate, enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten, herstellen.

Die folgenden Beispiele sollen die Herstellung der neuen Verbindungen verdeutlichen, ohne jedoch den Umfang auf die Einzelbeispiele zu beschränken. Die Verbindungen sind durch Analysen und durch spektroskopische Methoden (IR, NMR) in ihrer Struktur gesichert.

## Experimenteller Teil

### A. Herstellung von Ausgangsverbindungen

### Beispiele I bis XII

Allgemeine Arbeitsvorschrift für die Herstellung von Ketoalkoholen der allgemeinen Formel II.

Eine Mischung von 1,0 Mol eines Ketons der allgemeinen Formel VI mit 0,5 bis 4 Mol Formaldehyd in Form einer wäßrigen 35- bis 40%-igen Lösung wird mit Äthanol homogenisiert (20 bis 100 ml Äthanol) und mit methanolischer Kalilauge auf pH 10 bis 12 eingestellt. Die Mischung wird unter pH-Kontrolle mehrere Tage bei Raumtemperatur aufbewahrt. Ist das Keton laut Dünnschichtchromatographie weitgehend umgesetzt (nach ca. 2 bis 10 Tagen), wird mit Eisessig neutralisiert, anschließend das Lösungsmittel im Vakuum abdestilliert und der Rückstand unter vermindertem Druck destilliert. Gegebenenfalls wird die Reaktion bei geringerem Umsatz abgebrochen, wenn stärkere Nebenproduktbildung zu beobachten ist. (Bei der Destillation wurde in manchen Fällen, besonders wenn ein größerer Überschuß an Formaldehyd eingesetzt wurde, ein Ausgasen von Formaldehyd beobachtet.) Die Ausbeuten an Ketoalkoholen der Formel II liegen zwischen 50 und 90%.

Ketoalkohole, in denen beide A eine Brücke über den Sechsring bilden, werden im allgemeinen als Gemisch der exo/endo-Isomeren erhalten (lt. NMR-Spektroskopie: exo/endo = 9 : 1 bis 1 : 2), wobei sich die Bezeichnung exo und endo auf die Stellung der Gruppe $CH_2OH$ am überbrückten Sechsring bezieht. Die Gemische können durch Umkristallisation oder Säulenchromatographie getrennt werden (s. Beispiele XIII bis XV).

Beispiele I bis XII (Formel II) wurden nach dieser Vorschrift erhalten.

| Bsp. Nr. | A, A | Ringdoppelbindung, ggf. hydriert | $R^2$ | $R^3$ | $R^4$ | Sdp. [°C/Torr] bzw. Schmp. a) c) [°C] | | Analyse C | H | O |
|---|---|---|---|---|---|---|---|---|---|---|
| I | $—CH_2—$ | CH═CH | $CH_3$ | H | H | 105–107/0,1 | ber. | 72,25 | 8,49 | 19,25 |
| | | | | | | | gef. | 72,6 | 8,7 | 18,7 |
| | | | | | | | | $C_{10}H_{14}O_2$, M = 166 | | |
| II | $—CH_2—$ | CH═CH | $C_2H_5$ | H | H | 110–115/0,1 | ber. | 73,30 | 8,95 | 17,75 |
| | | | | | | | gef. | 73,1 | 8,8 | 17,7 |
| | | | | | | | | $C_{11}H_{16}O_2$, M = 180 | | |
| III | $—CH_2—$ | CH═CH | $iC_3H_7$ | H | H | 96– 97/0,1 | ber. | 74,19 | 9,34 | 16,47 |
| | | | | | | | gef. | 73,7 | 9,1 | 16,8 |
| | | | | | | | | $C_{12}H_{18}O_2$, M = 194 | | |
| IV | $—CH_2—$ | CH═CH | $CH_2CH(CH_3)_2$ | H | H | 120–121/0,1 | ber. | 75,0 | 9,6 | 15,3 |
| | | | | | | | gef. | 74,8 | 9,6 | 15,0 |
| | | | | | | | | $C_{13}H_{20}O_2$, M = 208 | | |
| V | $—CH_2—$ | $CH_2—CH_2$ | $CH_3$ | H | H | 104–108/2 | ber. | 71,39 | 9,59 | 19,02 |
| | | | | | | | gef. | 71,1 | 9,8 | 19,4 |
| | | | | | | | | $C_{10}H_{16}O_2$, M = 168 | | |
| VI | 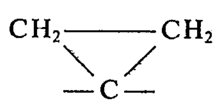 | CH═CH | $CH_3$ | H | H | 115–120/0,2 | | | | |
| VII | $—CH_2—CH_2—$ | CH═CH | $CH_3$ | H | H | 125–129/0,3 | ber. | 73,30 | 8,95 | 17,75 |
| | | | | | | | gef. | 73,6 | 8,6 | 17,7 |
| | | | | | | | | $C_{11}H_{16}O_2$, M = 180 | | |
| VIII | $CH_2—CH_2$ | $CH_2—CH_2$ | $CH_3$ | H | H | 43– 44 | ber. | 72,49 | 9,95 | 17,56 |
| | | | | | | | gef. | 72,4 | 9,7 | 17,7 |
| | | | | | | | | $C_{11}H_{18}O_2$, M = 182 | | |

Fortsetzung

| Bsp. Nr. | A, A | Ringdoppelbindung, ggf. hydriert | $R^2$ | $R^3$ | $R^4$ | Sdp. [°C/Torr] bzw. Schmp. a) c) [°C] | | Analyse C | H | O |
|---|---|---|---|---|---|---|---|---|---|---|
| IX | H,H | CH=CH | $CH_3$ | H | H | 127–131/0,2 | ber.<br>gef. | 70,10<br>70,8<br>$C_9H_{14}O_2$, M = 154 | 9,15<br>9,1 | 20,75<br>20,0 |
| X | H,H | CH=CH | $C_2H_5$ | H | H | 90–100/0,1[b]) | | | | |
| XI | H,H | C=C | $CH_3$ | 3-$CH_3$ | 4-$CH_3$ | 124–126/0,5 | ber.<br>gef. | 72,49<br>71,8<br>$C_{11}H_{18}O_2$, M = 182 | 9,95<br>9,6 | 17,56<br>18,3 |
| XII | H,H | $CH_2$—$CH_2$ | $CH_3$ | H | H | 110–113/0,1 | ber.<br>gef. | 69,2<br>68,8<br>$C_9H_{16}O_2$, M = 156 | 10,2<br>10,0 | 20,5<br>20,7 |

[a]) Zur Beachtung: Der tatsächliche Druck in der Destillationsapparatur ist aufgrund der Formaldehydabspaltung etwas höher als der an der Pumpe gemessene Druck.

[b]) Bei Darstellung dieses Ketoalkohols wurde neben der gewünschten Verbindung der stellungsisomere Ketoalkohol – Cyclohex-3'-en-1'-yl-1-hydroxyprop-2-yl-keton – gebildet. Die Trennung erfolgte durch Säulenchromatographie an Kieselgel mit Essigester/Cyclohexan 1 : 3 als Laufmittel.

[c]) exo/endo-Verhältnis (lt. NMR): Bsp. I ~ 5 : 1; Bsp. II–IV, VI ~ 1 : 1; Bsp. V, VII ~ 3 : 1–4 : 1.

Beispiele XIII bis XVII

Ketoalkohole der Formel II

## Beispiel XIII

exo-2-Hydroxymethylnorborn-5-en-2-yl-methylketon

250 g 2-Hydroxymethylnorborn-5-en-2-yl-methylketon aus Beispiel I werden mit wenig n-Hexan/Essigester bei Raumtemperatur versetzt. Beim Aufbewahren der Lösung im Kühlschrank fällt langsam eine kristalline Substanz aus, die abfiltriert (160 g vom Schmp. 34 bis 37°C) und in n-Hexan/Essigester umkristallisiert wird. Die laut Dünnschichtchromatographie (Laufmittel Essigester/Cyclohexan 1 : 1, Kieselgel-Platten) und NMR-Spektroskopie isomerenreine exo-Verbindung besitzt einen Schmelzpunkt von 44 bis 45°C. Durch Einengen der Mutterlaugen kann weiteres kristallines Material gewonnen werden. NMR ($CDCl_3$, 60 MHz): $\delta$= 1,2–1,9 ppm (m, 4 H); 2,0 (s, 3 H, $CH_3CO$), 2,5–3,0 (m, 2 H + OH); 3,75 (m, 2 H, $CH_2$–O); 5,75–6,2 (m, 2 H, –CH=CH–).

Analyse
ber. C 72,25 H 8,49 O 19,25%;
gef. C 72,1 H 8,4 O 19,6 %;
$C_{10}H_{14}O_2$, M = 166

## Beispiel XIV

endo-2-Hydroxymethyl-norborn-5-en-2-yl-methylketon

Die vereinigten Mutterlaugen aus der Gewinnung von exo-2-Hydroxymethyl-norborn-5-en-2-yl-keton (Beispiel XIII) werden an Kieselgel mit Essigester/Cyclohexan 1 : 3 chromatographiert. Man erhält lt. NMR-Spektren und Dünnschichtchromatographie reines endo-2-Hydroxymethyl-norborn-5-en-2-yl-methylketon als Öl.

NMR ($CDCl_3$, 60 MHz): 0,65–1,55 (m, 3 H); 1,88–2,2 (dd, 1 H); 2,18 (s, 3 H, $CH_3CO$); 2,6–3,2 (m, 2 H + OH); 3,45 (s, 2 H, $CH_2O$); 5,85–6,25 (m, 2 H, CH=CH).

## Beispiel XV

exo-2-Hydroxymethylnorborn-2-yl-methylketon

Analog Beispiel XIII konnte aus dem exo/endo-Gemisch aus Beispiel V durch Umkristallisation reines exo-2-Hydroxy-methyl-norborn-2-yl-methylketon vom Schmp. 48 bis 50°C erhalten werden.

Analyse
ber. C 71,39 H 9,59 O 19,02%;
gef. C 71,7 H 9,6 O 19,2 %;
$C_{10}H_{16}O_2$, M = 168

## Beispiel XVI

3,4-Dimethyl-1-hydroxymethyl-cyclohex-3-enyl-isobutyl-keton

Eine Mischung von 65,6 g (0,338 Mol) 3,4-Dimethylcyclohex-3-enyl-isobutylketon (Beispiel XXII), 120 ml Äthanol und 109,6 g 37%ige Formalinlösung (= 1,35 Mol) wird mit 15%iger methanolischer Kalilauge auf einen pH-Wert von pH 11 bis 12 eingestellt und 44 Stunden unter pH-Kontrolle am Rückfluß erhitzt. Anschließend wird die Lösung mit Eisessig auf pH 5 eingestellt und das Lösungsmittel im Vakuum abdestilliert. Der Rückstand wird mit Wasser versetzt und nach dem Sättigen mit Kochsalz fünfmal mit Toluol extrahiert. Nach dem Trocknen der organischen Phasen über getrocknetem Natriumsulfat wird das Lösungsmittel am Rotationsverdampfer abdestilliert und der Rückstand unter vermindertem Druck destilliert. Man erhält bei 112 bis 116°C/0,1 Torr Übergangstemperatur 37,7 g 3,4-Dimethyl-1-hydroxymethyl-cyclohex-3-enyl-isobutylketon.

## Beispiel XVII

### 2-Hydroxymethyl-bicyclo-[2.2.2]-oct-2-yl-methylketon

Eine Lösung von 10 g 2-Hydroxymethyl-bicyclo-[2.2.2]-oct-5-en-2-yl-methylketon (Beispiel VII) wird in 50 ml Methanol in Gegenwart von 1 g eines 1%igem Palladium/Kohle-Katalysators bei Raumtemperatur mit Wasserstoff bis zum Ende der Wasserstoffaufnahme hydriert. Nach dem Abfiltrieren des Katalysators wird das Lösungsmittel abdestilliert und der Rückstand (9,1 g = 90%) durch Säulenchromatographie an Kieselgel (Laufmittel Essigester/Cyclohexan 1 : 1) gereinigt. Man erhält reines 2-Hydroxy-methyl-bicyclo-[2.2.2]-octyl-methylketon vom Schmp. 43 bis 44°C (identisch mit Substanz aus Beispiel VIII).

## Beispiele XVIII bis XXI

Phenylcarbonate der allgemeinen Formel IV ($R^5 = OC_6H_5$).

## Beispiel XVIII

### (2-Acetylnorborn-5-en-2-yl)-methyl-phenylcarbonat (exo-Isomeres)

Eine Mischung von 265,6 g (1,6 Mol) exo-2-Hydroxymethylnorborn-5-en-2-yl-methylketon (Beispiel XIII), 400 ml absoluten Tetrahydrofuran, 342 g (1,6 Mol) Diphenylcarbonat und 242 g (2,4 Mol) Triäthylamin wird 8 Stunden bei leichtem Rückfluß gerührt. Nach dem Abdestillieren des Lösungsmittels unter vermindertem Druck wird der Rückstand in Äther aufgenommen und fünfmal mit 5%iger Natronlauge ausgezogen. Anschließend wird die organische Phase mit Wasser neutral gewaschen und über Natriumsulfat getrocknet.

Nach dem Abdestillieren des Lösungsmittels erhält man 418 g (= 91,2%) eines kristallinen Rückstandes, der aus 1500 ml Äther umkristallisiert wird. Man erhält 150 g (= 33%) (2-Acetyl-norborn-5-en-2-yl)-methyl-phenylcarbonat vom Schmp. 76 bis 77°C. Aus den Mutterlaugen wird weiteres Material erhalten.

Analyse
ber. C 71,3 H 6,3 O 22,4%;
gef. C 72,0 H 6,3 O 22,4%;
$C_{17}H_{18}O_4$, M = 286

## Beispiel XIX

### (2-Acetyl-bicyclo-[2.2.2]-oct-5-en-2-yl)-methyl-phenylcarbonat

Analog Beispiel XVIII erhält man aus 2-Hydroxymethyl-bicyclo-[2.2.2]-oct-5-en-2-yl-methylketon (Beispiel VII) (2-Acetyl-bicyclo-[2.2.2]-oct-5-en-2-yl)-methylphenylcarbonat vom Schmp. 58 bis 60°C.

Analyse
ber. C 71,98 H 6,71 O 21,31%;
gef. C 72,8 H 6,8 O 20,3 %;
$C_{18}H_{20}O_4$, M = 300

## Beispiel XX

### (1-Acetylcyclohex-3-en-yl)-methyl-phenylcarbonat

Analog Beispiel XVIII erhält man aus 1-Hydroxymethylcyclohex-3-enyl-methylketon (Beispiel IX) (1-Acetylcyclohex-3-enyl)-methyl-phenylcarbonat. Das Rohprodukt wird säulenchromatographisch gereinigt (Essigester/Cyclohexan 1 : 5 als Laufmittel; Kieselgel).

Analyse
ber. C 70,05 H 6,61 O 23,33%;
gef. C 69,9 H 6,8 O 23,2 %;
$C_{16}H_{18}O_4$, M = 274,3

Beispiel XXI

(2-Acetylbicyclo-[2.2.2]-octyl)-methyl-phenylcarbonat

Eine Mischung aus 11 g der Verbindung aus Beispiel XIX, 50 ml Methanol und 1 g eines Palladium/Kohle-Katalysators (10%Palladium) wird bei 20 bis 30°C bis zum Ende der Wasserstoffaufnahme hydriert (Dauer ca. 2,5 Stunden). Nach dem Abfiltrieren des Katalysators wird das Lösungsmittel unter vermindertem Druck abdestilliert und der Rückstand (9,3 g = 84%) aus Petroläther umkristallisiert. Man erhält 5,2 g (2-Acetylbicyclo-[2.2.2]-octyl)-methylphenylcarbonat vom Schmp. 74 bis 75°C.

Analyse
ber. C 71,49 H 7,33 O 21,16%;
gef. C 71,7 H 7,5 O 20,8 %;
$C_{18}H_{22}O_4$, M = 302,4

Beispiele XXII bis XXIV

Ketone der allgemeinen Formel VI

Die Ketone der allgemeinen Formel VI sind größtenteils literaturbekannt bzw. lassen sich nach literaturbekannten Verfahren, beispielsweise durch Diels-Alder-Reaktion, herstellen.

Beispiel XXII

3,4-Dimethylcyclohex-3-en-1-yl-isobutylketon

47,5 g 5-Methyl-hex-1-en-3-on werden bei 100°C mit 45,3 g 2,3-Dimethylbutadien versetzt, daß die Temperatur nicht unter 90°C sinkt. Nach 1 Stunde bei 100°C und 30 Minuten bei 115°C wird der Rückstand unter vermindertem Druck destilliert. Man erhält 64 g 3,4-Dimethylcyclohex-3-en-1-yl-isobutylketon vom Sdp. 99 bis 101°C/3 Torr.

Beispiel XXIII

7,7-(1,2-Äthyliden)norborn-5-en-2-yl-methylketon

20,5 g Spiro-[2,4]-hepta-3,5-dien wird auf 60°C erwärmt und mit 16,45 g Methylvinylketon versetzt. Man läßt noch 4 Stunden bei 60°C rühren und destilliert anschließend unter vermindertem Druck. Man erhält 26,6 g (= 76,4%) 7,7-(1,2-Äthyliden)-norborn-5-en-2-yl-methylketon vom Sdp. 90 bis 92°C/6 Torr.

Beispiel XXIV

Cyclohex-3-en-yl-äthylketon

In einer 250 ml Schüttelbombe läßt man 50 g Pent-1-en-3-on und 50 g Butadien in Gegenwart von 1 g Hydrochinon bei 100°C miteinander reagieren. Nach 5 Stunden wird abgekühlt und der Austrag destilliert. Man erhält 55 g = 76% Cyclohex-3-enyl-äthyl-keton vom Sdp. 78 bis 81°C/10 Torr.

B. Erfindungsgemäße Verbindungen

Beispiele 1 bis 77

Darstellung durch Umsetzung eines Ketoalkohols II mit einem Isocyanat III

Beispiel 1

2-exo-(N-Methylcarbamoyloxymethyl)-norborn-5-en-2-yl-methylketon

Eine Lösung von 16,6 g 2 exo-Hydroxymethylnorborn-5-en-2-yl-methylketon (Beispiel XIII) in 25 ml absolutem Tetrahydrofuran wird bei Raumtemperatur mit 6,3 g Methylisocyanat und 0,08 g Zinn-(II)-octanoat versetzt. Nach 20 Stunden wird das Lösungsmittel abdestilliert und der Rückstand (23 g = 100%) bei 170°C/0,01 Torr destilliert. Man erhält 13,8 g einer kristallinen Substanz vom Schmp. 51,5 bis 52,5°C.

Analyse
ber. C 64,57 H 7,62 N 6,28 O 21,52%;
gef. C 64,4 H 7,6 N 6,4 O 21,2 %;
$C_{12}H_{17}NO_3$, M = 223

Beispiele 2 bis 72

Allgemeine Arbeitsvorschrift für die Umsetzung eines Ketoalkohols der Formel II mit einem Isocyanat der Formel III

Zu einer Lösung von 0,05 Mol eines Ketoalkohols II in 10 bis 30 ml absolutem Tetrahydrofuran oder Äther werden bei Raumtemperatur 1 Gew.% Dibutylzinndiacetat — bezogen auf den eingesetzten Ketoalkohol — und anschließend 0,045 bis 0,055 Mol eines Isocyanats der Formel III ($R^1$ = H), das gegebenenfalls mit wenig THF oder Äther verdünnt wird, zugetropft. In vielen Fällen steigt die Temperatur der Reaktionsmischung um 10 bis 20°C an. Nach dem dünnschichtchromatographisch bestimmten Ende der Reaktion (Nachreaktionszeit bei +20 bis +40°C: 10 Minuten bis einige Stunden) wird das Lösungsmittel im Vakuum abgezogen und der Rückstand mit wenig Äther oder Essigester/Cyclohexan versetzt. Kristalline Produkte werden durch Umkristallisation (s. folgende Tabelle, 3. Spalte, U (= Umkristallisation) : Lösungsmittel), ölige Produkte durch Säulenchromatographie an Kieselgel (s. folgende Tabelle, 3. Spalte, SC (= Säulenchromatographie) : Laufmittel) gereinigt. In manchen Fällen kristallisieren die Produkte nach der Säulenchromatographie. Die Ausbeuten an reinem Produkt liegen im Bereich 50 bis 95%.

Die Verbindungen der folgenden Beispiele 2 bis 72 wurden nach dieser Vorschrift hergestellt.

Beispiele 2 bis 21

Umsetzung von exo-2-Hydroxymethylnorborn-5-en-2-yl-methylketon (Beispiel XIII) mit Isocyanaten $R^1NCO$

| Bsp. Nr. | $R^1$ | Reinigung des Rohproduktes | Schmp. [°C] | | Analyse C | H | N | O | Cl |
|---|---|---|---|---|---|---|---|---|---|
| 2 | $CH_3$ | U: Cyclohexan/Äther | 52–52,5 | identisch mit Substanz aus Beispiel 1 | | | | | |
| 3 | $C_2H_5$ | U: Äther | 41–42 | ber. | 65,8 | 8,1 | 5,9 | 20,1 | |
| | | | | gef. | 65,6 | 8,0 | 5,8 | 20,2 | |
| | | | | | $C_{13}H_{19}NO_3$, M = 237 | | | | |
| 4 | n-$C_3H_7$ | U: Äther + wenig Cyclohexan | 57–59 | ber. | 66,9 | 8,4 | 5,6 | 19,1 | |
| | | | | gef. | 67,1 | 8,4 | 5,4 | 19,0 | |
| | | | | | $C_{14}H_{21}NO_3$, M = 251 | | | | |
| 5 | n-$C_4H_9$ | U: Essigester/Hexan | 46–47 | ber. | 67,92 | 8,68 | 5,28 | 18,11 | |
| | | | | gef. | 67,8 | 8,5 | 5,8 | 18,2 | |
| | | | | | $C_{15}H_{23}NO_3$, M = 265,3 | | | | |
| 6 | n-$C_6H_{13}$ | U: n-Hexan | 35–36 | ber. | 69,6 | 9,2 | 4,8 | 16,4 | |
| | | | | gef. | 70,0 | 9,1 | 4,8 | | |
| | | | | | $C_{17}H_{27}NO_3$, M = 293 | | | | |
| 7 | i-$C_3H_7$ | U: Essigester/n-Hexan | 84–85 | ber. | 66,93 | 8,37 | 5,58 | 19,12 | |
| | | | | gef. | 66,8 | 8,3 | 6,1 | 19,1 | |
| | | | | | $C_{14}H_{21}NO_3$, M = 251,3 | | | | |
| 8 | t-$C_4H_9$ | U: Petroläther | 67–69 | ber. | 67,9 | 8,7 | 5,3 | 18,1 | |
| | | | | gef. | 67,8 | 8,8 | 5,4 | 18,1 | |
| | | | | | $C_{15}H_{23}NO_3$, M = 265 | | | | |
| 9 | $CH_2{=}CH-$ | U: Essigester | 65–66 | ber. | 66,4 | 7,3 | 6,0 | 20,4 | |
| | | | | gef. | 66,3 | 7,1 | 6,0 | 20,5 | |
| | | | | | $C_{13}H_{17}NO_3$, M = 235 | | | | |
| 10 | $CH_3-CH{=}CH-$ | U: Essigester/Cyclohexan 1 : 1 | 64–65 | | | | | | |

Fortsetzung

| Bsp. Nr. | $R^1$ | Reinigung des Rohproduktes | Schmp. [°C] | | Analyse C | H | N | O | Cl |
|---|---|---|---|---|---|---|---|---|---|
| 11 | $(CH_3)_2C{=}CH-$ | U: Essigester/n-Hexan | 76–78 | ber. | 68,42 | 8,04 | 5,32 | 18,23 | |
| | | | | gef. | 68,2 | 8,1 | 5,3 | 18,7 | |
| | | | | | $C_{15}H_{21}NO_3$, M = 263 | | | | |
| 12 | $CH_3OCH_2-$ | U: Cyclohexan + wenig Essigester | 51–52 | ber. | 61,6 | 7,6 | 5,5 | 25,3 | |
| | | | | gef. | 61,5 | 7,5 | 5,6 | 25,3 | |
| | | | | | $C_{13}H_{19}NO_4$, M = 253 | | | | |
| 13 | $CH_3SCH_2CH_2-$ | SC: Essigester/Cyclohexan 3 : 7 | Öl | ber. | 59,34 | 7,47 | 4,94 | 16,4 | |
| | | | | gef. | 58,7 | 7,3 | 4,9 | 17,5 | |
| | | | | | $C_{14}H_{21}NO_3S$, M = 283 | | | | |
| 14 | 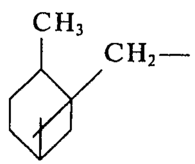 | SC: Essigester/Cyclohexan 3 : 7 | Öl | ber. | 73,5 | 9,3 | 3,9 | 13,4 | |
| | | | | gef. | 73,0 | 9,2 | 3,6 | 14,2 | |
| | | | | | $C_{22}H_{33}NO_3$, M = 360 | | | | |
| 15 | 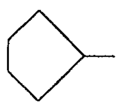 | U: Essigester | 98–99 | ber. | 69,3 | 8,4 | 5,0 | 17,3 | |
| | | | | gef. | 69,0 | 8,4 | 5,3 | 16,9 | |
| | | | | | $C_{16}H_{23}NO_3$, M = 277 | | | | |
| 16 | 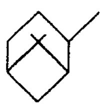 | SC: Essigester/Cyclohexan 1 : 1 | Öl | ber. | 72,5 | 8,8 | 4,2 | 14,5 | |
| | | | | gef. | 72,1 | 8,5 | 4,0 | 15,2 | |
| | | | | | $C_{20}H_{29}NO_3$, M = 331 | | | | |
| 17 | $C_6H_5-$ | U: Essigester/Hexan | 92–92,5 | ber. | 71,58 | 6,67 | 4,91 | 16,84 | |
| | | | | gef. | 71,5 | 6,3 | 5,4 | 16,9 | |
| | | | | | $C_{17}H_{19}NO_3$, M = 285 | | | | |
| 18 | $p\text{-}Cl-C_6H_4$ | U: Essigester | 149–151 | ber. | 63,8 | 5,6 | 4,4 | 15,0 | 11,1 |
| | | | | gef. | 63,7 | 5,7 | 4,7 | 14,7 | 11,3 |
| | | | | | $C_{17}H_{18}ClNO_3$, M = 319,5 | | | | |

Fortsetzung

| Bsp. Nr. | $R^1$ | Reinigung des Rohproduktes | Schmp. [°C] | | Analyse C | H | N | O | Cl |
|---|---|---|---|---|---|---|---|---|---|
| 19 | 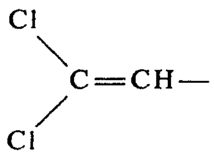 | U: Äther/n-Hexan | 80–81 | ber. | 51,33 | 4,97 | 4,60 | 15,78 | 23,31 |
| | | | | gef. | 51,5 | 5,1 | 4,8 | 15,8 | 23,2 |
| | | | | | $C_{13}H_{15}ClNO_3$, M = 304 | | | | |
| 20 | 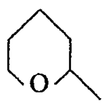 | U: Essigester/n-Hexan | 115–116 | ber. | 65,51 | 7,90 | 4,77 | 21,81 | |
| | | | | gef. | 65,1 | 7,8 | 5,1 | 21,8 | |
| | | | | | $C_{16}H_{23}NO_4$, M = 293 | | | | |
| 21 | 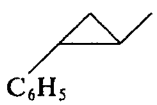 | SC: Essigester/Cyclohexan 3 : 7 | Öl | ber. | 73,82 | 7,12 | 4,30 | 14,75 | |
| | | | | gef. | 74,0 | 7,2 | 4,2 | 14,3 | |
| | | | | | $C_{20}H_{23}NO_3$, M = 325 | | | | |



## Beispiel 22

2-endo-(N-Isopropylcarbamoyloxymethyl)-norborn-5-en-2-yl-methylketon

Analog der allgemeinen Arbeitsvorschrift erhält man aus endo-2-Hydroxymethyl-norborn-5-en-2-yl-methylketon (Beispiel XIV) mit Isopropylisocyanat 2-endo-(N-Isopropylcarbamoyloxy-2-methyl)-norborn-5-en-2-yl-methylketon als Öl. Die Reinigung erfolgt durch Säulenchromatographie an Kieselgel mit Essigester/Cyclohexan 3 : 7 als Laufmittel.

Analyse
ber. C 66,93 H 8,37 N 5,58 O 19,12%;
gef. C 66,8 H 8,4 N 5,9 O 19,3 %;
$C_{14}H_{21}NO_3$, M = 251

Beispiele 23 bis 25

Umsetzung von exo-2-Hydroxymethyl-norborn-5-en-2-yl-äthylketon (Beispiel II) mit Isocyanaten $R^1NCO$

| Bsp. Nr. | $R^1$ | Reinigung des Rohproduktes | Schmp. [°C] | | Analyse C | H | N | O |
|---|---|---|---|---|---|---|---|---|
| 23 | $CH_3$ | SC: Essigester/Cyclohexan 1 : 3 | Öl | ber. | 65,80 | 8,07 | 5,90 | 20,23 |
| | | | | gef. | 65,6 | 7,9 | 6,0 | 20,3 |
| | | | | | $C_{13}H_{19}NO_3$, M = 237 | | | |
| 24 | $C_2H_5$ | SC: Essigester/Cyclohexan 1 : 5 | Öl | ber. | 66,91 | 8,42 | 5,57 | 19,10 |
| | | | | gef. | 66,4 | 8,2 | 5,5 | |
| | | | | | $C_{14}H_{21}NO_3$, M = 251 | | | |
| 25 | i-$C_3H_7$ | SC: Essigester/Cyclohexan 1 : 5 | Öl | ber. | 67,90 | 8,74 | 5,28 | 18,09 |
| | | | | gef. | 67,5 | 8,6 | 5,3 | 18,8 |
| | | | | | $C_{15}H_{23}NO_3$, M = 265 | | | |

Beispiele 26 bis 28

Umsetzung von 2-Hydroxymethyl-norborn-5-en-2-yl-isopropylketon (Beispiel III) mit Isocyanaten $R^1NCO$

| Bsp. Nr. | $R^1$ | Reinigung des Rohproduktes | Schmp. [°C] | | Analyse C | H | N | O |
|---|---|---|---|---|---|---|---|---|
| 26 | $CH_3$ | U: n-Hexan/Cyclohexan | 78–79 | ber. | 66,91 | 8,42 | 5,57 | 19,10 |
| | | | | gef. | 66,8 | 8,4 | 5,5 | 19,4 |
| | | | | | $C_{14}H_{21}NO_3$, M = 251 | | | |
| 27 | $C_2H_5$ | U: n-Hexan/Cyclohexan | 77–78 | ber. | 67,90 | 8,75 | 5,28 | 18,09 |
| | | | | gef. | 67,7 | 8,7 | 5,2 | 18,4 |
| | | | | | $C_{15}H_{23}NO_3$, M = 265 | | | |
| 28 | i-$C_3H_7$ | U: n-Hexan | 80–81 | ber. | 68,79 | 9,02 | 5,01 | 17,18 |
| | | | | gef. | 68,6 | 9,0 | 5,2 | 17,3 |
| | | | | | $C_{16}H_{25}NO_3$, M = 279 | | | |

## Beispiele 29 bis 31

Umsetzung von 2-Hydroxymethyl-norborn-5-en-2-yl-isobutylketon (Beispiel IV) mit Isocyanaten $R^1NCO$

| Bsp. Nr. | $R^1$ | Reinigung des Rohproduktes | Schmp. [°C] | | Analyse C | H | N | O |
|---|---|---|---|---|---|---|---|---|
| 29 | $CH_3$ | U: n-Hexan/Cyclohexan 1 : 1 | 78–79 | ber. | 67,90 | 8,74 | 5,28 | 18,09 |
| | | | | gef. | 67,9 | 8,7 | 5,5 | 17,8 |
| | | | | | $C_{15}H_{23}NO_3$, M = 265 | | | |
| 30 | $C_2H_5$ | U: n-Hexan/Cyclohexan 1 : 1 | 59–60 | ber. | 68,79 | 9,02 | 5,01 | 17,18 |
| | | | | gef. | 68,9 | 8,9 | 5,3 | 17,3 |
| | | | | | $C_{16}H_{25}NO_3$, M = 279 | | | |
| 31 | i-$C_3H_7$ | U: n-Hexan/Cyclohexan 1 : 1 | 70–71 | ber. | 69,59 | 9,28 | 4,77 | 16,36 |
| | | | | gef. | 69,3 | 9,3 | 5,3 | 16,2 |
| | | | | | $C_{17}H_{27}NO_3$, M = 293 | | | |

## Beispiele 32 bis 38

Umsetzung von exo-2-Hydroxymethylnorborn-2-yl-methylketon (Beispiel XV) mit Isocyanaten $R^1NCO$

| Bsp. Nr. | $R^1$ | Reinigung des Rohproduktes | Schmp. [°C] | | Analyse C | H | N | O |
|---|---|---|---|---|---|---|---|---|
| 32 | $CH_3$ | U: Essigester | 53–54 | ber. | 63,98 | 8,49 | 6,22 | 21,31 |
| | | | | gef. | 63,5 | 8,5 | 6,4 | 22,0 |
| | | | | | $C_{12}H_{19}NO_3$, M = 225 | | | |
| 33 | $C_2H_5$ | SC: Essigester/Cyclohexan 1 : 9 | 40–41 | ber. | 65,25 | 8,84 | 5,85 | 20,06 |
| | | | | gef. | 65,1 | 8,7 | 5,8 | |
| | | | | | $C_{13}H_{21}NO_3$, M = 239 | | | |
| 34 | n-$C_3H_7$ | U: Petroläther | 49–50 | ber. | 66,4 | 9,2 | 5,5 | 18,9 |
| | | | | gef. | 66,5 | 9,0 | 5,5 | 19,1 |
| | | | | | $C_{14}H_{23}NO_3$, M = 253 | | | |

Fortsetzung

| Bsp. Nr. | $R^1$ | Reinigung des Rohproduktes | Schmp. [°C] | | Analyse C | H | N | O |
|---|---|---|---|---|---|---|---|---|
| 35 | n-$C_4H_9$ | SC: Essigester/Cyclohexan 2 : 3 | Öl | ber.<br>gef. | 67,4<br>67,0<br>$C_{15}H_{25}NO_3$, M = 267 | 9,4<br>9,4 | 5,2<br>5,3 | 18,0 |
| 36 | i-$C_3H_7$ | U: Essigester | 79–80 | ber.<br>gef. | 66,4<br>66,1<br>$C_{14}H_{23}NO_3$, M = 253 | 9,2<br>9,1 | 5,5<br>5,9 | 18,9 |
| 37 | $CH_3OCH_2$— | 1) U: Hexan/Toluol<br>2) SC: Essigester/Cyclohexan 3 : 7 | 51–52 | ber.<br>gef. | 61,3<br>59,7<br>$C_{13}H_{21}NO_4$, M = 255 | 7,9<br>7,9 | 5,3<br>5,7 | 25,4 |
| 38 | 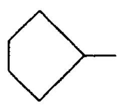 | U: Essigester | 92–93 | ber.<br>gef. | 68,8<br>68,8<br>$C_{16}H_{25}NO_3$, M = 279 | 9,0<br>8,9 | 5,0<br>5,1 | 17,2 |

Beispiele 39 bis 40

Umsetzung von Isocyanaten $R^1NCO$ mit 7,7-(1,2-Äthyliden)-2-hydroxymethylnorborn-5-en-2-yl-methylketon (Beispiel VI)

| Bsp. Nr. | $R^1$ | Reinigung des Rohproduktes | Schmp. [°C] | | Analyse C | H | N | O |
|---|---|---|---|---|---|---|---|---|
| 39 | $C_2H_5$ | SC: Essigester/Cyclohexan 3 : 7 | Öl | ber.<br>gef. | 68,42<br>69,3<br>$C_{15}H_{21}NO_3$, M = 263 | 8,04<br>8,1 | 5,32<br>5,1 | 18,23<br>18,4 |
| 40 | i-$C_3H_7$ | SC: Essigester/Cyclohexan 3 : 7 | Öl | ber.<br>gef. | 69,29<br>69,3<br>$C_{16}H_{23}NO_3$, M = 277 | 8,36<br>8,0 | 5,05<br>5,0 | 17,30<br>18,0 |

Beispiele 41 bis 45

Umsetzung von 2-Hydroxymethylbicyclo[2.2.2]oct-5-en-2-yl-methylketon (Beispiel VII) mit Isocyanaten $R^1NCO$

| Bsp. Nr. | $R^1$ | Reinigung des Rohproduktes | Schmp. [°C] | | Analyse C | H | N | O |
|---|---|---|---|---|---|---|---|---|
| 41 | $C_2H_5$ | SC: Essigester/Cyclohexan 1 : 7 | Öl | ber. | 66,91 | 8,42 | 5,57 | 19,10 |
| | | | | gef. | 66,7 | 8,5 | 5,6 | 19,2 |
| | | | | | $C_{14}H_{21}NO_3$, M = 251 | | | |
| 42 | n-$C_3H_7$ | SC: Essigester/Cyclohexan 1 : 5 | Öl | ber. | 67,90 | 8,74 | 5,28 | 18,09 |
| | | | | gef. | 67,7 | 8,8 | 5,7 | 18,4 |
| | | | | | $C_{15}H_{23}NO_3$, M = 265 | | | |
| 43 | n-$C_4H_9$ | SC: Essigester/Cyclohexan 1 : 7 | Öl | ber. | 68,79 | 9,02 | 5,01 | 17,18 |
| | | | | gef. | 68,5 | 8,8 | 5,1 | 17,1 |
| | | | | | $C_{16}H_{25}NO_3$, M = 279 | | | |
| 44 | i-$C_3H_7$ | U: Essigester | 78–80 | ber. | 67,90 | 8,74 | 5,28 | 18,09 |
| | | | | gef. | 67,5 | 9,2 | 5,6 | 17,9 |
| | | | | | $C_{15}H_{23}NO_3$, M = 265 | | | |
| 45 | $CH_3OCH_2$— | SC: Essigester/Cyclohexan 1 : 1,3 | 43–45 | ber. | 62,90 | 7,92 | 5,24 | 23,94 |
| | | | | gef. | 62,9 | 8,1 | 5,1 | 23,9 |
| | | | | | $C_{14}H_{21}NO_4$, M = 267 | | | |

Beispiele 46 bis 49

Umsetzung von 2-Hydroxymethylbicyclo[2.2.2]oct-2-yl-methylketon (Beispiel VIII) mit Isocyanaten $R^1CNO$

| Bsp. Nr. | $R^1$ | Reinigung des Rohproduktes | Schmp. [°C] | | Analyse C | H | N | O |
|---|---|---|---|---|---|---|---|---|
| 46 | $C_2H_5$ | U: Essigester/Petroläther | 60–61 | ber. | 66,37 | 9,15 | 5,53 | 18,95 |
| | | | | gef. | 66,5 | 8,9 | 5,7 | 19,1 |
| | | | | | $C_{14}H_{23}NO_3$, M = 253 | | | |

Fortsetzung

| Bsp. Nr. | $R^1$ | Reinigung des Rohproduktes | Schmp. [°C] | | Analyse C | H | N | O |
|---|---|---|---|---|---|---|---|---|
| 47 | $n\text{-}C_3H_7$ | U: Äther/n-Hexan | 55–56 | ber. | 67,38 | 9,42 | 5,24 | 17,95 |
| | | | | gef. | 67,2 | 9,4 | 5,3 | 18,1 |
| | | | | | $C_{15}H_{25}NO_3$, M = 267 | | | |
| 48 | $i\text{-}C_3H_7$ | U: Äther | 100–102 | ber. | 67,38 | 9,42 | 5,24 | 17,95 |
| | | | | gef. | 67,4 | 9,2 | 5,5 | 18,1 |
| | | | | | $C_{15}H_{25}NO_3$, M = 267 | | | |
| 49 | 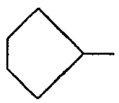 | U: Äther | 111–112 | ber. | 69,59 | 9,28 | 4,77 | 16,36 |
| | | | | gef. | 69,6 | 8,9 | 4,9 | 16,5 |
| | | | | | $C_{17}H_{27}NO_3$, M = 293 | | | |

Beispiele 50 bis 58

Umsetzung von 1-Hydroxymethyl-cyclohex-3-en-1-yl-methylketon (Beispiel IX) mit Isocyanaten $R^1NCO$

| Bsp. Nr. | $R^1$ | Reinigung des Rohproduktes | Schmp. [°C] | | Analyse C | H | N | O |
|---|---|---|---|---|---|---|---|---|
| 50 | $CH_3$ | SC: Essigester/Cyclohexan 1 : 1 | Öl | ber. | 62,5 | 8,1 | 6,6 | 22,7 |
| | | | | gef. | 61,9 | 8,0 | 6,7 | |
| | | | | | $C_{11}H_{17}NO_3$, M = 211 | | | |
| 51 | $C_2H_5$ | SC: Essigester/Cyclohexan 3 : 7 | Öl | ber. | 63,98 | 8,49 | 6,22 | 21,31 |
| | | | | gef. | 63,6 | 8,6 | 6,3 | 22,0 |
| | | | | | $C_{12}H_{19}NO_3$, M = 225 | | | |
| 52 | $n\text{-}C_3H_7$ | SC: Essigester/Cyclohexan 3 : 7 | Öl | ber. | 65,25 | 8,84 | 5,85 | 20,06 |
| | | | | gef. | 65,0 | 8,5 | 5,9 | 19,9 |
| | | | | | $C_{13}H_{21}NO_3$, M = 239 | | | |
| 53 | $n\text{-}C_4H_9$ | SC: Essigester/Cyclohexan 2 : 3 | Öl | ber. | 66,4 | 9,2 | 5,5 | 18,9 |
| | | | | gef. | 65,9 | 9,1 | 5,8 | |
| | | | | | $C_{14}H_{23}NO_3$, M = 253 | | | |

Fortsetzung

| Bsp. Nr. | $R^1$ | Reinigung des Rohproduktes | Schmp. [°C] | | Analyse C | H | N | O |
|---|---|---|---|---|---|---|---|---|
| 54 | $i\text{-}C_3H_7$ | SC: Essigester | Öl | ber. | 65,2 | 8,8 | 5,9 | 20,1 |
| | | | | gef. | 64,0 | 8,9 | 6,4 | |
| | | | | | $C_{13}H_{21}NO_3$, M = 239 | | | |
| 55 | $CH_3OCH_2$ | SC: Essigester/Cyclohexan 4 : 7 | Öl | ber. | 59,73 | 7,94 | 5,80 | 26,52 |
| | | | | gef. | 59,2 | 8,0 | 5,8 | |
| | | | | | $C_{12}H_{19}NO_4$, M = 241 | | | |
| 56 | $CH_2{=}CH-$ | SC: Essigester/Cyclohexan 3 : 7 | Öl | ber. | 64,55 | 7,67 | 6,27 | 21,50 |
| | | | | gef. | 64,3 | 7,6 | 6,3 | |
| | | | | | $C_{12}H_{17}NO_3$, M = 223 | | | |
| 57 | 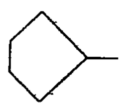 | SC: Essigester/Cyclohexan 3 : 7 | Öl | ber. | 67,9 | 8,7 | 5,2 | 18,1 |
| | | | | gef. | 67,3 | 8,9 | 4,9 | |
| | | | | | $C_{15}H_{23}NO_3$, M = 265 | | | |
| 58 | 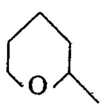 | SC: Essigester/Cyclohexan 3 : 7 | Öl | ber. | 64,04 | 8,24 | 4,98 | 22,75 |
| | | | | gef. | 63,4 | 8,1 | 4,9 | 23,0 |
| | | | | | $C_{15}H_{23}NO_4$, M = 281 | | | |

## Beispiel 59

1-(N-Isopropylcarbamoyloxymethyl)cyclohex-3-enyl-äthylketon

Aus 1-Hydroxymethylcyclohex-3-enyl-äthylketon (Beispiel X) wird durch Umsetzung mit Isopropylisocyanat 1-(N-Isopropylcarbamoyloxymethyl)cyclohex-3-enyl-äthylketon als Öl erhalten. Die Reinigung erfolgt durch Säulenchromatographie an Kieselgel mit Essigester/Cyclohexan 1 : 5.

Analyse
ber. C 66,37 H 9,15 N 5,53 O 18,95%;
gef. C 65,9 H 8,9 N 5,5 O 19,6 %;
$C_{14}H_{23}NO_3$, M = 253

Beispiele 60 bis 65

Umsetzung von 3,4-Dimethyl-1-hydroxymethylcyclohex-3-enyl-methylketon (Beispiel XI) mit Isocyanaten $R^1NCO$

| Bsp. Nr. | $R^1$ | Reinigung des Rohproduktes | Schmp. [°C] | | Analyse C | H | N | O |
|---|---|---|---|---|---|---|---|---|
| 60 | $C_2H_5$ | SC: Essigester/Cyclohexan 2 : 5 | Öl | ber. | 66,37 | 9,15 | 5,53 | 18,95 |
| | | | | gef. | 66,1 | 9,1 | 5,3 | 19,5 |
| | | | | | $C_{14}H_{23}NO_3$, M = 253 | | | |
| 61 | n-$C_3H_7$ | SC: Essigester/Cyclohexan 1 : 3 | Öl | ber. | 67,38 | 9,42 | 5,24 | 17,95 |
| | | | | gef. | 66,5 | 9,1 | 5,3 | 18,8 |
| | | | | | $C_{15}H_{25}NO_3$, M = 267 | | | |
| 62 | n-$C_4H_9$ | SC: Essigester/Cyclohexan 1 : 7 | Öl | ber. | 68,29 | 9,67 | 4,98 | 17,06 |
| | | | | gef. | 67,9 | 9,4 | 5,4 | 16,9 |
| | | | | | $C_{16}H_{27}NO_3$, M = 281 | | | |
| 63 | i-$C_3H_7$ | SC: Essigester/Cyclohexan 1 : 7 | Öl | ber. | 68,29 | 9,67 | 4,98 | 17,06 |
| | | | | gef. | 67,9 | 9,4 | 5,4 | 16,9 |
| | | | | | $C_{15}H_{25}NO_3$, M = 281 | | | |
| 64 | $CH_3OCH_2$— | SC: Essigester/Cyclohexan 1 : 1,3 | Öl | ber. | 62,43 | 8,61 | 5,20 | 23,76 |
| | | | | gef. | 62,0 | 8,4 | 5,4 | 23,8 |
| | | | | | $C_{14}H_{23}NO_4$, M = 269 | | | |
| 65 | 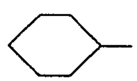 | SC: Essigester/Cyclohexan 1 : 4 | Öl | ber. | 70,32 | 9,51 | 4,56 | 15,61 |
| | | | | gef. | 70,3 | 9,3 | 4,6 | 15,9 |
| | | | | | $C_{18}H_{29}NO_3$, M = 307 | | | |

## Beispiele 66 bis 67

Umsetzung von 3,4-Dimethyl-1-hydroxymethylcyclohex-3-enyl-isobutylketon (Beispiel XVI) mit Isocyanaten $R^1NCO$

| Bsp. Nr. | $R^1$ | Reinigung des Rohproduktes | Schmp. [°C] | | Analyse C | H | N | O |
|---|---|---|---|---|---|---|---|---|
| 66 | $C_2H_5$ | SC: Essigester/Cyclohexan 1 : 6 | Öl | ber. | 67,07 | 9,93 | 4,60 | 18,40 |
| | | | | gef. | 67,1 | 9,4 | 4,4 | 18,7 |
| | | | | | $C_{17}H_{29}NO_3 \cdot 1/2\ H_2O$, M = 304,4 | | | |
| 67 | i-$C_3H_7$ | SC: Essigester/Cyclohexan 1 : 6 | Öl | ber. | 69,86 | 10,10 | 4,53 | 15,51 |
| | | | | gef. | 69,1 | 9,7 | 4,5 | |
| | | | | | $C_{18}H_{31}NO_3$, M = 309 | | | |

## Beispiele 68 bis 72

Umsetzung von 1-Hydroxymethylcyclohexyl-methylketon (Beispiel XII) mit Isocyanaten $R^1NCO$

| Bsp. Nr. | $R^1$ | Reinigung des Rohproduktes | Schmp. [°C] | | Analyse C | H | N | O |
|---|---|---|---|---|---|---|---|---|
| 68 | $CH_3$ | SC: Essigester/Cyclohexan 1 : 6 | Öl | ber. | 61,95 | 8,95 | 6,57 | 22,50 |
| | | | | gef. | 62,0 | 9,0 | 6,9 | 22,9 |
| | | | | | $C_{11}H_{19}NO_3$, M = 213 | | | |
| 69 | $C_2H_5$ | SC: Essigester/Cyclohexan 1 : 5 | Öl | ber. | 63,41 | 9,31 | 6,16 | 21,12 |
| | | | | gef. | 63,5 | 9,1 | 6,0 | 21,5 |
| | | | | | $C_{12}H_{21}NO_3$, M = 227 | | | |
| 70 | n-$C_4H_9$ | SC: Essigester/Cyclohexan 1 : 3 | Öl | ber. | 65,85 | 9,87 | 5,49 | 18,80 |
| | | | | gef. | 65,7 | 9,5 | 5,7 | 19,3 |
| | | | | | $C_{14}H_{25}NO_3$, M = 255 | | | |
| 71 | i-$C_3H_7$ | U: Cyclohexan | 51–52 | ber. | 64,70 | 9,61 | 5,80 | 19,89 |
| | | | | gef. | 64,9 | 9,4 | 6,2 | 19,8 |
| | | | | | $C_{13}H_{23}NO_3$, M = 241 | | | |
| 72 | t-$C_4H_9$ | U: Essigester/n-Hexan | 98–99 | ber. | 65,85 | 9,87 | 5,49 | 18,80 |
| | | | | gef. | 65,7 | 9,4 | 5,4 | 18,8 |
| | | | | | $C_{14}H_{25}NO_3$, M = 255 | | | |

Beispiele 73 bis 77

Umsetzung von Cyansäure mit Ketoalkoholen der allgemeinen Formel II.

Beispiel 73

2-Carbamoyloxymethyl-norborn-5-en-2-yl-methylketon

Zu einer Lösung von 66,4 g (0,4 Mol) 2-Hydroxymethylnorborn-5-en-2-yl-methylketon in 120 ml Methylenchlorid gibt man bei 20°C unter langsamem Rühren 52 g (0,8 Mol) Natriumcyanat und anschließend tropfenweise 95,8 g (0,84 Mol) Trifluoressigsäure zu. Bei Raumtemperatur wird 5 Stunden nachgerührt und anschließend mit Wasser verdünnt. Nach mehrmaliger Extraktion mit Methylenchlorid werden die organischen Phasen vereinigt und über Natriumsulfat getrocknet. Nach dem Abdestillieren des Lösungsmittels erhält man einen kristallinen Rückstand (83,1 g = 99%), der in Essigester umkristallisiert wird. Man erhält 45,8 g 2-Carbamoyloxymethyl-norborn-5-en-2-y-methylketon vom Schmp. 118 bis 119°C.

Analyse
ber. C 63,1 H 7,2 N 6,7 O 22,9%;
gef. C 63,3 H 7,1 N 6,8%;
$C_{11}H_{15}NO_3$, M = 209

Beispiele 74 bis 77

Analog Beispiel 73 werden aus dem jeweils entsprechenden Ketoalkohol der Formel II die folgenden Carbamate hergestellt:

Beispiel 74

2-Carbamoyloxymethylnorborn-5-en-2-yl-isopropylketon

Schmp.: 131 bis 133°C (Umkristallisation aus Essigester)

Analyse
ber. C 65,80 H 8,07 N 5,90 O 20,23%;
gef. C 65,5 H 7,9 N 6,1 O 20,1 %;
$C_{13}H_{19}NO_3$, M = 237

Beispiel 75

2-Carbamoyloxymethyl-norborn-5-en-2-yl-isobutylketon

Schmp.: 81 bis 83°C (Umkristallisation aus Essigester/Hexan)

Analyse
ber. C 66,4 H 8,9 N 5,5 O 19,0%;
gef. C 66,7 H 8,3 N 5,7 O 19,1%;
$C_{14}H_{23}NO_3$, M = 253

Beispiel 76

1-Carbamoyloxymethyl-3,4-dimethylcyclohex-3-enyl-methylketon

Schmp.: 110 bis 111°C (Umkristallisation aus Petroläther/Essigester)

Analyse
ber. C 63,98 H 8,50 N 6,22 O 21,31%;
gef. C 64,0 H 8,2 N 6,4 O 21,6 %;
$C_{12}H_{19}NO_3$, M = 225

Beispiel 77

1-Carbamoyloxymethyl-3,4-dimethylcyclohex-3-enyl-isobutylketon

Schmp.: 128 bis 130°C (Umkristallisation aus Essigester/n-Hexan)

Analyse
ber. C 67,38 H 9,42 N 5,24 O 17,95%;
gef. C 67,4 H 9,3 N 5,2 O 18,14%;
$C_{15}H_{25}NO_3$, M = 267

Beispiele 78 bis 128

Allgemeine Arbeitsvorschrift zur Umsetzung von Phenylcarbonaten der allgemeinen Formel IV ($R^5 = OC_6H_5$) mit Aminen $R^1NH_2$

Zu einer Lösung von 0,033 Mol eines Phenylcarbonats der Formel IV ($R^5 = OC_6H_5$) in 5 bis 30 ml Tetrahydrofuran oder Äther werden bei Raumtemperatur 0,033 Mol bis 0,04 Mol Amin $R^1NH_2$ pur, als Lösung in Tetrahydrofuran oder Äther oder besonders bei tiefsiedenden Aminen als wäßrige Lösung zugetropft. Nach dem dünnschichtchromatographisch bestimmten Ende der Reaktion (5 Minuten bis 2 Stunden) wird gegebenenfalls Tetrahydrofuran durch Äther ersetzt und die organische Phase zwei- bis fünfmal mit 5%iger Natronlauge ausgezogen und einmal mit gesättigter Kochsalzlösung gewaschen. Nach Trocknen der organischen Phase über Natriumsulfat und Abziehen des Lösungsmittels erhält man, gegebenenfalls nach Behandlung mit wenig Lösungsmittel, wie z. B. Essigester/Cyclohexan, in vielen Fällen kristalline Rohprodukte, die aus einem geeigneten Lösungsmittel umkristallisiert werden. Ölige Rohprodukte werden durch Säulenchromatographie über Kieselgel gereinigt. Die Ausbeuten an reinem Produkt liegen im Bereich 50 bis 95%.

Beispiele 78 bis 118

Umsetzung von (2-Acetylnorborn-5-en-2-yl)-methyl-phenylcarbonat (Beispiel XVIII) mit Aminen $R^1NH_2$

| Bsp. Nr. | $R^1$ | Reinigung des Rohproduktes | Schmp. [°C] | | Analyse C | H | N | O |
|---|---|---|---|---|---|---|---|---|
| 78 | H | U: Essigester | 118–119 | | identisch mit Substanz aus Beispiel 73 | | | |
| 79 | $C_2H_5$ | U: Äther | 41– 42 | | identisch mit Substanz aus Beispiel 3 | | | |
| 80 | n-$C_3H_7$ | U: Äther/Cyclohexan | 57– 59 | | identisch mit Substanz aus Beispiel 4 | | | |
| 81 | n-$C_4H_9$ | U: Essigester/Hexan | 46– 47 | | identisch mit Substanz aus Beispiel 5 | | | |
| 82 | n-$C_5H_{11}$ | U: n-Hexan | 49– 50 | ber. | 68,8 | 9,0 | 5,0 | 17,2 |
| | | | | gef. | 68,9 | 9,2 | 5,0 | |
| | | | | | $C_{16}H_{25}NO_3$, M = 279 | | | |
| 83 | n-$C_6H_{13}$ | U: n-Hexan | 35– 36 | | identisch mit Substanz aus Beispiel 6 | | | |
| 84 | n-$C_8H_{17}$ | U: n-Hexan | 49– 50 | ber. | 71,0 | 9,6 | 4,4 | 14,9 |
| | | | | gef. | 71,2 | 9,5 | 4,4 | |
| | | | | | $C_{19}H_{31}NO_3$, M = 321 | | | |
| 85 | n-$C_{12}H_{25}$ | U: n-Hexan | 70– 71 | ber. | 73,2 | 10,4 | 3,7 | 12,7 |
| | | | | gef. | 73,4 | 10,2 | 3,7 | |
| | | | | | $C_{23}H_{39}NO_3$, M = 377 | | | |
| 86 | i-$C_3H_7$ | U: Essigester/n-Hexan | 84– 85 | | identisch mit Substanz aus Beispiel 7 | | | |
| 87 | $(CH_3)_2CH-CH_2-$ | U: Cyclohexan | 55– 57 | ber. | 67,9 | 8,7 | 5,3 | 18,1 |
| | | | | gef. | 68,0 | 8,7 | 5,4 | |
| | | | | | $C_{15}H_{23}NO_3$, M = 265 | | | |
| 88 | $CH_3-CH_2-CH(CH_3)-$ | U: Cyclohexan | 81– 82 | ber. | 67,9 | 8,7 | 5,3 | 18,1 |
| | | | | gef. | 67,8 | 8,4 | 5,1 | 18,7 |
| | | | | | $C_{15}H_{23}NO_3$, M = 265 | | | |

Fortsetzung

| Bsp. Nr. | $R^1$ | Reinigung des Rohproduktes | Schmp. [°C] | | Analyse C | H | N | O |
|---|---|---|---|---|---|---|---|---|
| 89 | $(CH_3)_2CH-CH(CH_3)-$ | SC: Essigester/Cyclohexan 1 : 3 | 80– 81 | ber.<br>gef. | 68,8<br>69,0<br>$C_{16}H_{25}NO_3$, M = 279 | 9,0<br>8,8 | 5,0<br>5,0 | 17,2<br>17,1 |
| 90 | $CH_2{=}CH-CH_2$ | U: Cyclohexan/Essigester | 51– 52 | ber.<br>gef. | 67,45<br>67,0<br>$C_{14}H_{19}NO_3$, M = 249 | 7,68<br>7,2 | 5,62<br>5,9 | 19,25 |
| 91 | $CH_2{=}C(CH_3)-CH_2$ | U: Hexan/Essigester | 36– 37 | ber.<br>gef. | 68,4<br>68,4<br>$C_{15}H_{21}NO_3$, M = 263 | 8,0<br>8,0 | 5,3<br>5,2 | 18,2 |
| 92 | $HC{\equiv}C-CH_2$ | U: Cyclohexan/Essigester | 48– 50 | ber.<br>gef. | 68,00<br>68,0<br>$C_{14}H_{17}NO_3$, M = 247 | 6,93<br>7,0 | 5,66<br>5,7 | 19,41 |
| 93 | $NC-CH_2-CH_2-$ | SC: Essigester/Cyclohexan 1 : 3 | Öl | ber.<br>gef. | 64,11<br>63,9<br>$C_{14}H_{18}N_2O_3$, M = 262 | 6,92<br>6,9 | 10,68<br>10,8 | 18,30<br>18,7 |
| 94 | $CH_3OCH_2CH(CH_3)-$ | SC: Essigester/Cyclohexan 1 : 3 | Öl | ber.<br>gef. | 64,04<br>64,2<br>$C_{15}H_{23}NO_4$, M = 281 | 8,24<br>8,1 | 4,98<br>4,9 | 22,74<br>23,2 |
| 95 | $(CH_3)_2CHO(CH_2)_3-$ | U: Petroläther/Äther | 42– 43 | ber.<br>gef. | 65,99<br>66,2<br>$C_{17}H_{27}NO_4$, M = 309 | 8,80<br>8,7 | 4,53<br>4,5 | 20,68<br>20,7 |
| 96 | $(CH_3O)_2CH-CH(CH_3)-$ | SC: Essigester/Cyclohexan 1 : 3 | Öl | ber.<br>gef. | 61,72<br>61,5<br>$C_{16}H_{25}NO_5$, M = 311 | 8,09<br>7,8 | 4,50<br>4,4 | 25,68<br>25,8 |
| 97 | 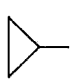 $-CH_2-$ | U: Äther/n-Hexan | 66– 67 | ber.<br>gef. | 68,42<br>68,3<br>$C_{15}H_{21}NO_3$, M = 263 | 8,04<br>7,7 | 5,32<br>5,3 | 18,23<br>18,6 |

Fortsetzung

| Bsp. Nr. | $R^1$ | Reinigung des Rohproduktes | Schmp. [°C] | | Analyse C | H | N | O | |
|---|---|---|---|---|---|---|---|---|---|
| 98 | 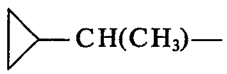 | U: Cyclohexan | 72 – 73 | ber.<br>gef. | 69,1<br>69,5<br>$C_{16}H_{23}NO_3$, M = 277 | 8,6<br>8,5 | 5,0<br>5,0 | 17,3<br>17,3 | |
| 99 | 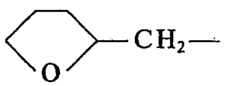 | U: Äther | 44 – 45 | ber.<br>gef. | 65,51<br>65,3<br>$C_{16}H_{23}NO_4$, M = 293 | 7,90<br>7,7 | 4,77<br>4,8 | 21,80<br>21,8 | |
| 100 | 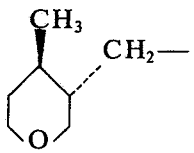 | U: Hexan/Essigester | 80 – 81 | ber.<br>gef. | 67,27<br>67,1<br>$C_{18}H_{27}NO_4$, M = 321 | 8,47<br>8,4 | 4,36<br>4,6 | 19,90<br>20,3 | |
| 101 | 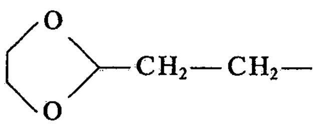 | SC: Essigester/Cyclohexan | Öl | ber.<br>gef. | 62,12<br>61,9<br>$C_{16}H_{23}NO_5$, M = 309 | 7,49<br>7,5 | 4,53<br>4,5 | 25,86<br>26,0 | |
| 102 | 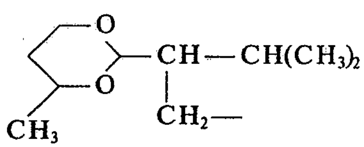 | SC: Essigester/Cyclohexan 1 : 3 | Öl | ber.<br>gef. | 66,46<br>66,7<br>$C_{21}H_{33}NO_5$, M = 351,4 | 8,77<br>8,7 | 3,69<br>3,7 | 21,08<br>21,2 | |
| 103 | $C_6H_5CH_2$— | U: Äther | 56 – 57 | ber.<br>gef. | 72,2<br>72,1<br>$C_{18}H_{21}NO_3$, M = 299 | 7,1<br>7,2 | 4,7<br>4,8 | 16,0 | |
| 104 | 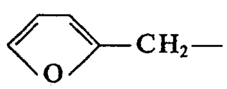 | U: Äther | 70 – 71 | ber.<br>gef. | 66,4<br>66,3<br>$C_{16}H_{19}NO_4$, M = 289 | 6,6<br>6,6 | 4,8<br>4,9 | 22,2 | |
| 105 | 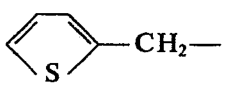 | U: Äther | 60 – 61 | ber.<br>gef. | 62,93<br>63,3<br>$C_{16}H_{19}NO_3S$, M = 305 | 6,27<br>6,2 | 4,59<br>4,7 | 15,72<br>16,1 | 10,50 |

Fortsetzung

| Bsp. Nr. | $R^1$ | Reinigung des Rohproduktes | Schmp. [°C] | | Analyse C | H | N | O |
|---|---|---|---|---|---|---|---|---|
| 106 | $C_6H_5—CH(CH_3)—CH_2—$ | SC: Essigester/Cyclohexan 3 : 7 | Öl | ber. | 73,4 | 7,7 | 4,3 | 14,7 |
| | | | | gef. | 72,8 | 7,6 | 4,2 | |
| | | | | | $C_{20}H_{25}NO_3$, M = 327 | | | |
| 107 | 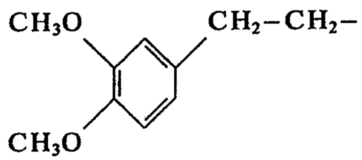 | U: Äther/Essigester | 82– 83 | ber. | 67,5 | 7,2 | 3,8 | 21,5 |
| | | | | gef. | 67,4 | 7,1 | 3,6 | |
| | | | | | $C_{21}H_{27}NO_5$, M = 373 | | | |
| 108 | $(C_6H_5)_2CH—CH_2CH_2—$ | SC: Essigester/Cyclohexan 2 : 5 | 80– 81 | ber. | 77,39 | 7,24 | 3,47 | 11,89 |
| | | | | gef. | 77,2 | 7,4 | 3,6 | 12,1 |
| | | | | | $C_{26}H_{29}NO_3$, M = 403 | | | |
| 109 | 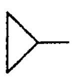 | U: Cyclohexan/Essigester | 57– 58 | ber. | 67,4 | 7,7 | 5,6 | 19,3 |
| | | | | gef. | 67,6 | 7,6 | 5,6 | |
| | | | | | $C_{14}H_{19}NO_3$, M = 249 | | | |
| 110 | 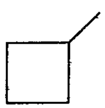 | U: Cyclohexan | 70– 71 | ber. | 68,42 | 8,04 | 5,32 | 18,23 |
| | | | | gef. | 68,6 | 8,0 | 5,3 | 18,2 |
| | | | | | $C_{15}H_{21}NO_3$, M = 263 | | | |
| 111 | 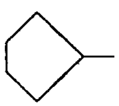 | U: Essigester | 98– 99 | | identisch mit Substanz aus Beispiel 15 | | | |
| 112 | 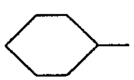 | U: Essigester | 108–109 | ber. | 70,1 | 8,6 | 4,8 | 16,5 |
| | | | | gef. | 69,8 | 8,3 | 4,5 | |
| | | | | | $C_{17}H_{25}NO_3$, M = 291 | | | |
| 113 | 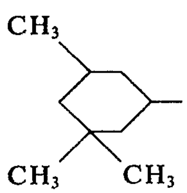 | U: Äther | 104–105 | ber. | 72,04 | 9,37 | 4,20 | 14,39 |
| | | | | gef. | 71,9 | 9,4 | 4,3 | 14,5 |
| | | | | | $C_{20}H_{31}NO_3$, M = 333 | | | |

Fortsetzung

| Bsp. Nr. | $R^1$ | Reinigung des Rohproduktes | Schmp. [°C] | | Analyse C | H | N | O |
|---|---|---|---|---|---|---|---|---|
| 114 | 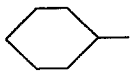 | U: Äther | 115–116 | ber. | 70,79 | 8,91 | 4,59 | 15,72 |
| | | | | gef. | 70,8 | 8,7 | 4,6 | 16,0 |
| | | | | | $C_{18}H_{27}NO_3$, M = 305 | | | |
| 115 | 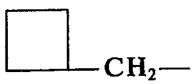 | SC: Essigester/Cyclohexan 1 : 5 | 75– 76 | ber. | 69,29 | 8,36 | 5,05 | 17,30 |
| | | | | gef. | 69,2 | 8,1 | 4,7 | 18,2 |
| | | | | | $C_{16}H_{23}NO_3$, M = 277 | | | |
| 116 | 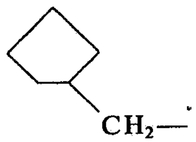 | U: Essigester | 78– 80 | ber. | 70,07 | 8,65 | 4,81 | 16,47 |
| | | | | gef. | 69,9 | 8,7 | 5,6 | 16,2 |
| | | | | | $C_{17}H_{25}NO_3$, M = 291 | | | |
| 117 | 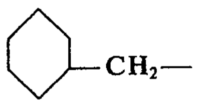 | SC: Essigester/Cyclohexan 1 : 7 | 78– 80 | ber. | 70,79 | 8,91 | 4,59 | 15,72 |
| | | | | gef. | 70,4 | 8,5 | 4,5 | 16,0 |
| | | | | | $C_{18}H_{27}NO_3$, M = 305 | | | |
| 118 | 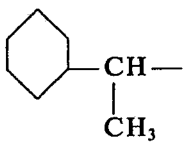 | SC: Essigester/Cyclohexan 1 : 7 | 94– 96 | ber. | 71,44 | 9,15 | 4,38 | 15,03 |
| | | | | gef. | 71,4 | 8,9 | 3,8 | 15,4 |
| | | | | | $C_{19}H_{29}NO_3$, M = 319 | | | |

Beispiele 119 bis 126

Umsetzung von (1-Acetylcyclohex-3-enyl)methyl-phenylcarbonat (Beispiel XX) mit einem Amin $R^1NH_2$

| Bsp. Nr. | $R^1$ | Reinigung des Rohproduktes | Schmp. [°C] | | Analyse C | H | N | O |
|---|---|---|---|---|---|---|---|---|
| 119 | n-$C_3H_7$ | SC: Essigester/Cyclohexan 3 : 7 | Öl | | identisch mit Substanz aus Beispiel 52 | | | |
| 120 | n-$C_4H_9$ | SC: Essigester/Cyclohexan 2 : 3 | Öl | | identisch mit Substanz aus Beispiel 53 | | | |
| 121 | n-$C_5H_{11}$ | SC: Essigester/Cyclohexan 1 : 5 | Öl | ber. | 67,38 | 9,42 | 5,24 | 17,95 |
| | | | | gef. | 66,9 | 9,4 | 5,3 | 18,4 |
| | | | | | $C_{15}H_{25}NO_3$, M = 267 | | | |
| 122 | n-$C_6H_{13}$ | SC: Essigester/Cyclohexan 1 : 5 | Öl | ber. | 68,29 | 9,67 | 4,98 | 17,06 |
| | | | | gef. | 67,9 | 9,5 | 5,0 | 16,9 |
| | | | | | $C_{16}H_{27}NO_3$, M = 281 | | | |
| 123 | i-$C_3H_7$ | SC: Essigester/Cyclohexan 1 : 1 | Öl | | identisch mit Substanz aus Beispiel 54 | | | |
| 124 | $CH_3-CH_2-CH(CH_3)-$ | SC: Essigester/Cyclohexan 1 : 5 | 45–46 | ber. | 66,37 | 9,15 | 5,53 | 18,95 |
| | | | | gef. | 66,6 | 8,8 | 5,3 | 19,1 |
| | | | | | $C_{14}H_{23}NO_3$, M = 253 | | | |
| 125 | 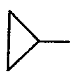 $-CH(CH_3)-$ | SC: Essigester/Cyclohexan 1 : 7 | 73–75 | ber. | 67,92 | 8,68 | 5,28 | 18,11 |
| | | | | gef. | 67,0 | 8,5 | 5,0 | 18,8 |
| | | | | | $C_{15}H_{23}NO_3$, M = 265 | | | |
| 126 | 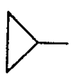 | SC: Essigester/Cyclohexan 1 : 5 | Öl | ber. | 65,80 | 8,07 | 5,90 | 20,23 |
| | | | | gef. | 65,8 | 8,1 | 5,7 | 20,8 |
| | | | | | $C_{13}H_{19}NO_3$, M = 237,3 | | | |

Beispiel 127

2-(N-Cyclopentylcarbamoyloxymethyl)bicyclo[2.2.2]oct-5-en-2-yl-methylketon

Analog der allgemeinen Arbeitsvorschrift wird aus (2-Acetylbicyclo[2.2.2]oct-5-en-2-yl)methyl-phenylcarbonat (Beispiel XIX) durch Umsetzung mit Cyclopentylamin 2-(N-Cyclopentylcarbamoyloxy-methyl)bicyclo[2.2.2]oct-5-en-2-yl-methylketon hergestellt. Das Rohprodukt wird aus Äther umkristallisiert. Schmp. 77 bis 78°C.

Analyse

| | | | | |
|---|---|---|---|---|
| ber. | C 70,07 | H 8,65 | N 4,81 | O 16,47%; |
| gef. | C 70,2 | H 8,6 | N 5,0 | O 16,5 %; |

$C_{17}H_{25}NO_3$, M = 291

Beispiel 128

2-(N-Cyclopentylcarbamoyloxymethyl)bicyclo[2.2.2]oct-2-yl-methylketon

Analog der allgemeinen Arbeitsvorschrift wird aus Cyclopentylamin und (2-Acetylbicyclo[2.2.2]oct-2-yl)methyl-phenylcarbonat (Beispiel XXI) 2-(N-Cyclopentylcarbamoyloxymethyl)bicyclo[2.2.2]oct-2-yl-methylketon mit Schmp. 111 bis 112°C (Umkristallisation aus Äther) erhalten (identisch mit Substanz aus Beispiel 49).

C. Beispiele 129 bis 133

Allgemeine Arbeitsvorschrift zur Hydrierung der Ringdoppelbindung von Carbamaten der Formel I

Eine Lösung von 50 mMol eines ungesättigten Carbamats der Formel I, in 50 ml Methanol wird in Gegenwart von 0,1 bis 1 g Palladium/Kohle (10% Palladiumgehalt) bei Raumtemperatur bis zur Aufnahme der theoretischen Wasserstoffmenge, bzw. bis zum Ende der Wasserstoffaufnahme hydriert. Nach dem Abfiltrieren des Katalysators wird das Lösungsmittel unter vermindertem Druck abdestilliert und der Rückstand umkristallisiert oder säulenchromatographisch gereinigt. Man erhält mit 60 bis 95% Ausbeute gesättigte Carbamate der Formel I.

Nach dieser Vorschrift werden die Verbindungen der Beispiele 129 bis 133 (Formel I, $R^2 = CH_3$; $R^3$, $R^4 = H$) hergestellt.

| Bsp. Nr. | Ausgangs-substanz Bsp. Nr. | A, A | $R^1$ | Reinigung | Schmp. [°C] | Identisch mit Substanz aus Beispiel |
|---|---|---|---|---|---|---|
| 129 | 7 | $-CH_2-$ | i-$C_3H_7$ | SC: Essigester/Cyclohexan 1 : 3 | 79–80 | 36 |
| 130 | 41 | $-CH_2-CH_2-$ | $C_2H_5$ | U: Essigester/Petroläther | 60–61 | 46 |
| 131 | 42 | $-CH_2-CH_2-$ | n-$C_3H_7$ | U: Äther/n-Hexan | 55–56 | 47 |
| 132 | 54 | H, H | i-$C_3H_7$ | SC: Essigester/Cyclohexan 1 : 1 | 46–47 | 71 |
| 133 | 53 | H, H | n-$C_4H_9$ | SC: Essigester/Cyclohexan 1 : 1 | Öl | 70 |

## Beispiel 134

### 2-[N-(4-Methoxy-3-methylphenyl)carbamoyloxymethyl]-norborn-5-en-2-yl-methylketon

Eine Lösung von 10,54 g (0,077 Mol) 4-Methoxy-3-methylanilin in 30 ml THF wird mit 2,1 g 80% NaH-Suspension versetzt und zur Vervollständigung der Wasserstoffentwicklung auf 40°C erwärmt. Nach Ende der Gasabspaltung wird bei Raumtemperatur eine Lösung von 20,0 g (0,077 Mol) (2-Acetylnorborn-5-en-2-yl)methylphenylcarbonat in 20 ml Tetrahydrofuran zugetropft und die Mischung 18 Stunden bei 20°C gerührt.

Nach Zusatz von wenig Wasser wird das Lösungsmittel abgezogen und der Rückstand in Äther aufgenommen. Die Ätherphase wird mehrmals mit 5%iger Natronlauge und mit Wasser gewaschen und über Natriumsulfat getrocknet. Das kristalline Rohprodukt (11,8 g) wird aus Essigester umkristallisiert; 6,4 g 2-[N-(4-Methoxy-3-methylphenyl)carbamoyloxymethyl]-norborn-5-en-2-yl-methylketon vom Schmp. 121 bis 122°C.

Analyse
ber. C 69,28 H 7,04 N 4,25 O 19,4%;
gef. C 69,4 H 6,9 N 4,3%;
$C_{19}H_{23}NO_4$, $M = 329$

## Beispiele 135 bis 137

### Allgemeine Arbeitsvorschrift zur Umsetzung von (2-Acetylnorborn-5-en-2-yl)methyl-phenylcarbonat mit Aminhydrohalogeniden (in situ-Freisetzung der Amine)

In eine Lösung von 6,0 g (0,021 Mol) (2-Acetylnorborn-5-en-2-yl)methylphenylcarbonat in 30 ml absoluten Methanol wurden bei 20°C zuerst 0,023 Mol Aminhydrohalogenid ($R^1NH_2 \cdot HX$, $X = Cl, Br$) und anschließend während 2 Stunden eine Lösung von 1,1 g (0,021 Mol) Natriummethylat in 20 ml Methanol zugetropft. Man läßt noch 15 bis 60 Minuten bei Raumtemperatur nachreagieren und zieht das Lösungsmittel im Vakuum ab. Der Rückstand wird mit Wasser verdünnt und mit Äther mehrmals extrahiert. Anschließend werden die vereinigten organischen Phasen dreimal mit 5%iger Natronlauge und einmal mit Wasser ausgeschüttelt, über Natriumsulfat getrocknet und am Rotationsverdampfer eingeengt und über Kieselgel säulenchromatographiert.

Die Ausbeuten an reinen Produkten lagen im Bereich 30 bis 50%, bezogen auf eingesetztes (2-Acetylnorborn-5-en-2-yl)-methyl-phenylcarbonat. Durch Verwendung von 2 bis 4 Äquivalenten Aminhydrohalogenid und Natriummethylat ließ sich die Ausbeute auf über 50% steigern.

Beispiele 135 bis 137

| Bsp. Nr. | $R^1$ | Reinigung des Rohproduktes | Schmp. [°C] | | Analyse C | H | N | O | Hal |
|---|---|---|---|---|---|---|---|---|---|
| 135 | $ClCH_2CH_2$— | SC: Essigester/Cyclohexan 1 : 4 | 74–75 | ber. | 57,46 | 6,68 | 5,15 | 17,66 | Cl 13,05 |
| | | | | gef. | 57,6 | 6,7 | 5,0 | 17,5 | Cl 13,0 |
| | | | | | $C_{13}H_{18}ClNO_3$, M = 272 | | | | |
| 136 | $Cl-CH_2-CH_2-CH_2-$ | SC: Essigester/Cyclohexan 1 : 4 | 41–43 | ber. | 58,84 | 7,05 | 4,90 | 16,80 | Cl 12,41 |
| | | | | gef. | 59,0 | 7,1 | 4,7 | 17,0 | Cl 12,4 |
| | | | | | $C_{14}H_{20}ClNO_3$, M = 286 | | | | |
| 137 | $Br-CH_2-CH_2-CH_2-$ | SC: Essigester/Methylenchlorid 3 : 7 | Öl | ber. | 50,92 | 6,10 | 4,24 | 14,54 | Br 24,20 |
| | | | | gef. | 51,0 | 6,1 | 4,3 | 15,5 | Br 23,7 |
| | | | | | $C_{14}H_{20}BrNO_3$, M = 330 | | | | |

Beispiel 138

1-(N-Isopropylcarbamoyloxymethyl)-cyclohex-3-enyl-methylketon

In eine Lösung von 12,5 g Phosgen in 60 ml absolutem Äther wird bei +5°C eine Lösung von 10,0 g 1-Hydroxymethylcyclohex-3-enyl-methylketon (Beispiel IX) in 10 ml Äther eingetropft. Nach dreistündigem Rühren bei Raumtemperatur wird das überschüssige Phosgen und das Lösungsmittel am Rotationsverdampfer abdestilliert und der Rückstand [Chlorkohlensäure-(1-acetylcyclohex-3-enyl)-methyl-ester] in 100 ml abs. Toluol aufgenommen und mit 17,7 g Isopropylamin versetzt. Dabei steigt die Temperatur auf +40°C an, anschließend wird die Mischung 3 Stunden bei 80°C nachgerührt und nach dem Abkühlen auf Raumtemperatur in 150 ml Wasser gegossen. Nach dem Abtrennen der organischen Phase wird die wäßrige Phase zweimal mit Toluol extrahiert. Die vereinigten Toluolphasen werden einmal mit Wasser gewaschen und über Natriumsulfat getrocknet. Nach dem Abdestillieren des Lösungsmittels wird das Rohprodukt säulenchromatographisch gereinigt (Laufmittel Essigester/Cyclohexan 1 : 4). Man erhält 10,7 g NMR-spektroskopisch reines 1-(N-Isopropylcarbamoyloxymethyl)-cyclohex-3-enyl-methyl-keton, das mit dem Produkt aus Beispiel 54 identisch ist. Daneben werden 1,8 g des eingesetzten Ketoalkohols (Beispiel IX) zurückgewonnen. Die Ausbeute, bezogen auf nicht zurückgewonnenen Ketoalkohol, beträgt 84%.

Analog dieser Vorschrift werden aus dem entsprechenden Chlorkohlensäureester der Formel IV, der in situ aus einem Ketoalkohol der Formel II (Beispiel XIII, IX und XV), mit Phosgen hergestellt wird, mit vergleichbaren Ausbeuten erhalten.

exo-2-(N-Isopropylcarbamoyloxymethyl)-norborn-5-en-2-yl-methylketon
exo-2-(N-n-Propylcarbamoyloxymethyl)-norborn-5-en-2-yl-methylketon
exo-2-(N-Cyclopentylcarbamoyloxymethyl)-norborn-5-en-2-yl-methylketon
exo-2-(N-Isopropylcarbamoyloxymethyl)-norborn-2-yl-methylketon
exo-2-(N-Cyclopropylcarbamoyloxymethyl)-norborn-5-en-2-yl-methylketon
exo-2-[N-(2-Methylpropyl)-carbamoyloxymethyl]-norborn-5-en-2-yl-methylketon
exo-2-(N-But-2-ylcarbamoyloxymethyl)-norborn-5-en-2-yl-methylketon
1-(N-Propylcarbamoyloxymethyl)-cyclohex-3-en-yl-methylketon
exo-2-[N-(1-Cyclopropyläthyl)-carbamoyloxymethyl]-norborn-5-en-2-yl-methylketon

Beispiel 139

40 g eines Gemisches aus Norbonenylmethylketon und 2-Hydroxymethylnorborn-5-en-2-yl-methylketon (exo-endo-Verhältnis ca. 2 : 8) werden in 64 ml Tetrahydrofuran mit 22,4 g Isopropylisocyanat in Gegenwart von 0,4 g Dibutylzinndiacetat umgesetzt. Nach dem Abklingen der exothermen Reaktion (Erwärmung bis auf 50°C) wird 3 Stunden lang bei Raumtemperatur nachgerührt und das Lösungsmittel abdestilliert. Man erhält 65 g eines gelben Öls, das zum großen Teil kristallisiert. Nach Umkristallisation aus Cyclohexan/Hexan (1 : 1) erhält man 35,5 g 2-(N-Isopropyl-carbamoyloxymethyl)-norborn-5-en-2-yl-methylketon vom Schmelzpunkt 78 bis 79°C. Die weitere Umkristallisation liefert 26,4 g eines exo-2-(N-Isopropyl-carbamoyloxymethyl)-norborn-5-en-2-yl-methylketon vom Schmelzpunkt 84°C.

D. Formulierungsbeispiele, die in üblicher Weise hergestellt werden

1. Tabletten

| | | |
|---|---|---|
| a) | Ein Wirkstoff der Formel I | 50 mg |
| | Lactose | 200 mg |
| | Methylcellulose | 15 mg |
| | Maisstärke | 50 mg |
| | Talkum | 11 mg |
| | Magnesiumstearat | 4 mg |
| | | 330 mg |
| b) | Ein Wirkstoff der Formel I | 20 mg |
| | Lactosek | 173 mg |
| | Avicel | 80 mg |
| | Polywachs 6000 | 20 mg |
| | Magnesiumstearat | 2 mg |
| | | 300 mg |

c)

| | |
|---|---|
| Verbindung der Formel I | 250 mg |
| Polyvinylpyrrolidon (mittl. M. G. 25 000) | 170 mg |
| Polyäthylenglykol (mittl. M. G. 4 000) | 14 mg |
| Hydroxypropylmethylcellulose | 40 mg |
| Talkum | 4 mg |
| Magnesiumstearat | 2 mg |
| | 480 mg |

Der Wirkstoff wird mit Polyvinylpyrrolidon in 10%iger wäßriger Lösung befeuchtet, durch ein Sieb mit der lichten Maschenweite 1,0 mm getrieben und bei 50°C getrocknet. Dieses Granulat wird mit Polyäthylenglykol (mittl. M. G. 4000), Hydroxypropylmethylcellulose, Talkum und Magnesiumstearat vermischt und zu Tabletten a 280 mg verpreßt.

Beispiel für Dragees

| | |
|---|---|
| Verbindung der Formel I | 50 mg |
| Lactose | 90 mg |
| Maisstärke | 60 mg |
| Polyvinylpyrrolidon | 6 mg |
| Magnesiumstearat | 1 mg |
| | 207 mg |

Die Mischung der Wirkstoffsubstanz mit Lactose und Maisstärke wird mit einer 8%igen wäßrigen Lösung des Polyvinylpyrrolidons durch Sieb 1,5 mm granuliert, bei 50°C getrocknet und nochmals durch Sieb 1,0 mm getrieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

3. Kapselformulierung

| | |
|---|---|
| Verbindung der Formel I | 50 mg |
| Magnesiumstearat | 2 mg |
| Milchzucker | 30 mg |
| | 82 mg |

4. Injektionslösung

| | |
|---|---|
| Verbindung der Formel I | 100 mg |
| Natriumchlorid | 9 mg |
| destilliertes Wasser, q. s. auf 10 ml | |

**Patentansprüche**

1. Verbindungen der Formel I

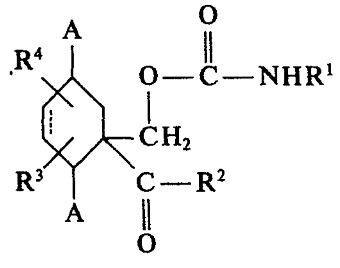

(I)

in denen die gestrichelte Linie eine Doppelbindung, die gegebenenfalls hydriert ist, bedeutet und die Reste A für Wasserstoffatome stehen oder beide Reste A zusammen eine Brücke der Formel

$$—(CH_2)_m—,$$

in der m für die Zahlen 1 oder 2 steht, bilden und $R^1$ einen gesättigten geradkettigen oder verzweigten Alkylrest mit 1 bis 6 Kohlenstoffatomen, der gegebenenfalls durch ein Halogenatom oder einen Niedrigalkoxyrest mit 1 bis 3 Kohlenstoffatomen, eine Cyanogruppe oder einen Cycloalkylrest mit 3 oder 4 Kohlenstoffatomen im Ring substituiert sein kann, einen Alkenyl- oder Alkinylrest mit 2 bis 6 Kohlenstoffatomen oder einen Cycloalkylrest mit 3 bis 6 Kohlenstoffatomen im Ring, $R^2$ Methyl oder Ethyl, $R^3$ und $R^4$ Wasserstoffatome oder Methylgruppen bedeuten.

2. Verbindungen der Formel I nach Anspruch 1, in denen die gestrichelte Linie eine Doppelbindung, die gegebenenfalls hydriert ist, bedeutet und die Reste A für Wasserstoffatome stehen oder beide Reste A zusammen einen Methylenrest bilden und $R^1$ einen gesättigten, geradkettigen oder verzweigten Alkylrest mit 1 bis 6 Kohlenstoffatomen, wobei ein Alkylrest mit 1 bis 3 Kohlenstoffatomen gegebenenfalls durch ein Chloratom, eine Methoxygruppe oder eine Cycloalkylgruppe mit 3 bis 4 Kohlenstoffatomen im Ring substituiert sein kann, einen Alkenyl- oder Alkinylrest mit 2 bis 4 Kohlenstoffatomen oder einen Cycloalkylrest mit 3 bis 5 Kohlenstoffatomen im Ring, $R^2$ Methyl und $R^3$ und $R^4$ Wasserstoffatome bedeuten.

3. exo-2-(N-Isopropylcarbamoyloxymethyl)-norborn-5-en-2-yl-methylketon.

4. exo-2-(N-n-Propylcarbamoyloxymethyl)-norborn-5-en-2-yl-methylketon.

5. exo-2-(N-Cyclopentylcarbamoyloxymethyl)-norborn-5-en-2-yl-methylketon.

6. exo-2-(N-Isopropylcarbamoyloxymethyl)-norborn-2-yl-methylketon.

7. exo-2-(N-Cyclopropylcarbamoyloxymethyl)-norborn-5-en-2-yl-methylketon.

8. exo-2-[N-(2-Methylprop-1-yl)-carbamoyloxymethyl]-norborn-5-en-2-yl-methylketon.

9. exo-2-(N-But-2-ylcarbamoyloxymethyl)-norborn-5-en-2-yl-methylketon.

10. 1-(N-Propylcarbamoyloxymethyl)-cyclohex-3-enyl-methylketon.

11. exo-2-[N-(1-Cyclopropyläthyl)-carbamoyloxymethyl]-norborn-5-en-2-yl-methylketon.

12. Verfahren zur Herstellung von Verbindungen der Formel (I) nach Anspruch 1, dadurch gekennzeichnet, daß man

a) einen β-Ketoalkohol der allgemeinen Formel II,

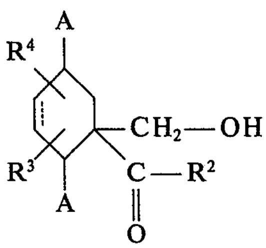

(II)

in der die gestrichelte Linie eine Doppelbindung, die gegebenenfalls hydriert sein kann, bedeutet und die Reste A und $R^2$ bis $R^4$ die für Formel I angegebenen Bedeutungen haben, mit einem Isocyanat der allgemeinen Formel III,

$$R^1{-}N{=}C{=}O \qquad (III)$$

in der $R^1$ die für Formel I angegebenen Bedeutungen hat, oder in Form seines Säureadditionsadduktes in Gegenwart einer Base bei Temperaturen von 0 bis 140°C, zweckmäßigerweise in einem inerten Lösungsmittel und gegebenenfalls in Anwesenheit eines Katalysators umsetzt, wobei eine erhaltene ungesättigte Verbindung gegebenenfalls anschließend katalytisch hydriert wird oder

b) einen β-Ketoalkohol der allgemeinen Formel II in Form eines Kohlensäureesters der Formel IV

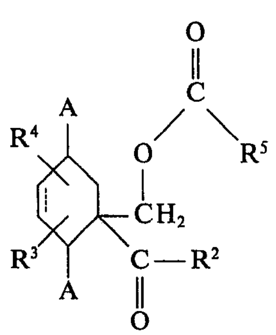

(IV)

in der A und $R^2$ bis $R^4$ die für Formel I angegebenen Bedeutungen haben und $R^5$ ein Halogenatom, vorzugsweise ein Chloratom, oder den Rest –OAr, wobei Ar eine gegebenenfalls substituierte Phenylgruppe darstellt, mit einem Amin der allgemeinen Formel V

$R^1—NH_2$ (V)

in der $R^1$ die für Formel I angegebenen Bedeutungen hat mit der Maßgabe, daß der Rest $R^1$ keine zum Stickstoffatom $\alpha,\beta$-ständige Doppel- oder Dreifachbindung aufweist, bei Temperaturen von 0 bis 120°C, zweckmäßigerweise in einem Lösungsmittel und gegebenenfalls in Anwesenheit eines basischen Katalysators umsetzt, wobei eine erhaltene ungesättigte Verbindung gegebenenfalls anschließend katalytisch hydriert wird.

13. Pharmazeutisches Mittel, enthaltend eine Verbindung der Formel (I) nach Anspruch 1 als Wirkstoff neben üblichen Trägerstoffen und Verdünnungsmitteln.

14. Eine Verbindung der Formel (I) nach Anspruch 1 zur Verwendung als Hypnotikum bei der Behandlung von Schlafstörungen oder als Sedativum.

## Claims

1. A compound of the formula I

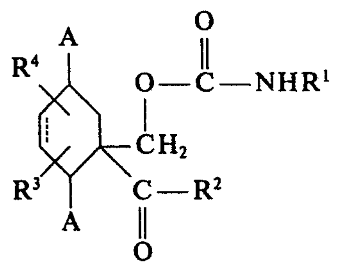

(I)

where the broken line is a double bond which may or may not be hydrogenated, and the radicals A are each hydrogen, or the two radicals A together are a bridge of the formula

$$—(CH_2)_m—,$$

where m is one of the integers 1 and 2, and $R^1$ is a saturated straight-chain or branched alkyl radical of 1 to 6 carbon atoms, which is optionally substituted by halogen, lower alkoxy of 1 to 3 carbon atoms, cyano, or cycloalkyl of 3 or 4 carbon atoms in the ring; alkenyl or alkynyl of 2 to 6 carbon atoms; or cycloalkyl of 3 to 6 carbon atoms in the ring, $R^2$ is methyl or ethyl, and $R^3$ and $R^4$ are each hydrogen or lower alkyl.

2. A compound of the formula I as claimed in claim 1, where the broken line is a double bond, which may or may not be hydrogenated, and the radicals A are each hydrogen or together are methylene, and $R^1$ is saturated, straight-chain or branched alkyl of 1 to 6 carbon atoms, or alkyl of 1 to 3 carbon atoms which is substituted by chlorine, methoxy, or cycloalkyl of 3 or 4 carbon atoms, or is alkenyl or alkynyl of 2 to 4 carbon atoms or is cycloalkyl of 3 to 5 carbon atoms in the ring, $R^2$ is methyl and $R^3$ and $R^4$ are each hydrogen.

3. exo-2-(N-Isopropylcarbamyloxymethyl)-norborn-5-en-2-yl-methyl-ketone.

4. exo-2-(N-n-Propylcarbamyloxymethyl)-norborn-5-en-2-yl-methyl-ketone.

5. exo-2-(N-Cyclopentylcarbamyloxymethyl)-norborn-5-en-2-yl-methyl-ketone.

6. exo-2-(N-Isopropylcarbamyloxymethyl)-norborn-2-yl-methyl-ketone.

7. exo-2-(N-Cyclopropylcarbamyloxymethyl)-norborn-5-en-2-yl-methyl-ketone.

8. exo-2-[N-(2-Methylprop-1-yl)-carbamyloxymethyl]-norborn-5-en-2-yl-methyl-ketone.

9. exo-2-(N-But-2-ylcarbamyloxymethyl)-norborn-5-en-2-yl-methyl-ketone.

10. 1-(N-Propylcarbamyloxymethyl)-cyclohex-3-enyl-methyl-ketone.

11. exo-2-[N-(1-Cyclopropylethyl)-carbamyloxymethyl)-norborn-5-en-2-yl-methyl-ketone.

12. A process for the preparation of a compound of the formula (I) as claimed in claim 1, wherein

a) a $\beta$-keto-alcohol of the general formula II

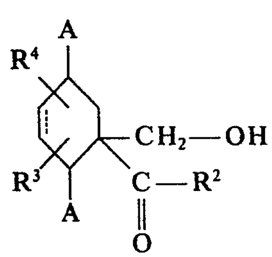

(II)

where the broken line is a double bond, which may or may not be hydrogenated, and the radicals A, $R^2$, $R^3$ and $R^4$ have the meanings given for formula I, is reacted with an isocyanate of the general formula III

$$R^1—N=C=O \qquad (III)$$

where $R^1$ has the meanings given for formula I, or with an acid adduct thereof in the presence of a base, at from 0 to 140°C, advantageously in an inert solvent, in the presence or absence of a catalyst, with or without subsequent catalytic hydrogenation if the compound obtained is unsaturated; or

b) a β-keto-alcohol of the general formula II, in the form of a carbonic acid ester of the formula IV

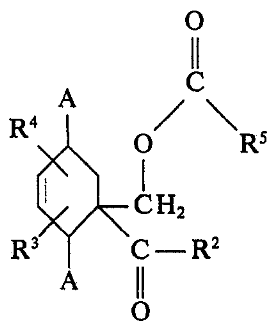

(IV)

where A, $R^2$, $R^3$ and $R^4$ have the meanings given for formula I and $R^5$ is halogen, preferably chlorine, or is –OAr, Ar being unsubstituted or substituted phenyl, is reacted with an amine of the general formula V

$$R^1—NH_2 \qquad (V)$$

where $R^1$ has the meanings given for formula I, with the proviso that $R^1$ does not contain a double or triple bond in the β-position to the nitrogen, at from 0 to 120°C, advantageously in a solvent, in the presence or absence of a basic catalyst, with or without subsequent catalytic hydrogenation if the compound obtained is unsaturated.

13. A pharmaceutical agent containing a compound of the formula I as claimed in claim 1, as the active compound, in addition to conventional carriers and diluents.

14. A compound of the formula (I), as claimed in claim 1, for use as a hypnotic in the treatment of sleep disturbance, or as a sedative.

## Revendications

1. Composés de la formule I

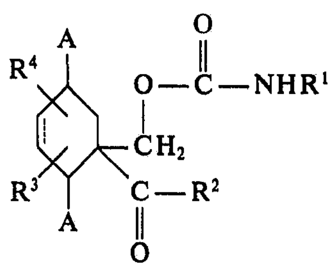

(I)

dans laquelle le trait interrompu représente une double liaison éventuellement hydrogénée, les restes A désignent des atomes d'hydrogène ou forment ensemble un pont de la formule

$$—(CH_2)_m—,$$

où m vaut 1 ou 2, $R^1$ désigne un radical alkyle en $C_1$ à $C_8$ saturé à chaîne droite ou ramifiée, éventuellement substitué par un atome d'halogène, un radical alcoxy inférieur en $C_1$ à $C_3$, un groupe cyano ou un groupe cycloalkyle avec un noyau cyclique avec trois ou quatre atomes de carbone, un radical alcényle ou alcynyle en $C_2$ à $C_6$ ou un groupe cycloalkyle avec un noyau cyclique comprenant trois à six atomes de carbone, $R^2$ désigne un radical méthyle ou éthyle et $R^3$ et $R^4$ représentent des atomes d'hydrogène ou

des radicaux méthyle.

2. Composés de la formule I selon la revendication 1, pour lesquels le trait interrompu représente une double liaison éventuellement hydrogénée, les restes A désignent des atomes d'hydrogène ou forment ensemble un pont méthylène, $R^1$ désigne un radical alkyle en $C_1$ à $C_8$ saturé à chaîne droite ou ramifiée, un radical alkyle en $C_1$ à $C_3$ éventuellement substitué par un atome de chlore, un groupe méthoxy ou un groupe cycloalkyle avec un noyau cyclique comportant trois ou quatre atomes de carbone, un radical alcényle ou alcynyle en $C_2$ à $C_4$ ou un groupe cycloalkyle avec un noyau cyclique avec trois à cinq atomes de carbone, $R^2$ = méthyle et $R^3 = R^4 = H$.

3. La exo-2-(N-isopropylcarbamoyloxyméthyl)-norborn-5-én-2-yl-méthyl-cétone.

4. La exo-2-(N-n-propylcarbamoyloxyméthyl)-norborn-5-én-2-yl-méthyl-cétone.

5. La exo-2-(N-cyclopentylcarbamoyloxyméthyl)-norborn-5-én-2-yl-méthyl-cétone.

6. La exo-2-(N-isopropylcarbamoyloxyméthyl)-norborn-5-én-2-yl-méthyl-cétone.

7. La exo-2-(N-cyclopropylcarbamoyloxyméthyl)-norborn-5-én-2-yl-méthyl-cétone.

8. La exo-2-[N-(2-méthyl-propyl-1)-carbamoyloxyméthyl]-norborn-5-én-2-yl-méthyl-cétone.

9. La exo-2-(N-butyl-2-carbamoyloxyméthyl)-norborn-5-én-2-yl-méthyl-cétone.

10. La 1-(N-propylcarbamoyloxyméthyl)-cyclohex-3-ényl-méthyl-cétone.

11. La exo-2-[N-(1-cyclopropyléthyl)-carbamoyloxyméthyl]-norborn-5-én-2-yl-méthyl-cétone.

12. Procédé de préparation de composés de la formule (I) selon la revendication 1, caractérisé en ce que l'on fait réagir:

a) un β-céto-alcool de la formule générale II

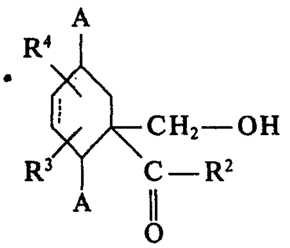

(II)

dans laquelle le trait interrompu représente une double liaison éventuellement hydrogénée et les restes A et $R^2$ à $R^4$ possèdent les significations définies pour la formule I, avec un isocyanate de la formule générale III

$$R^1{-}N{=}C{=}O \qquad (III)$$

dans laquelle $R^1$ possède les significations définies pour la formule I, ou avec un sel d'addition d'un acide d'un tel composé, généralement dans un solvant inerte, à des températures de 0 à 140°C, en présence d'une base et le cas échéant d'un catalyseur, un composé non saturé obtenu pouvant être soumis ensuite à une hydrogénation catalysée; ou

b) un β-céto-alcool de la formule générale II sous forme d'un ester d'acide carbonique de la formule IV

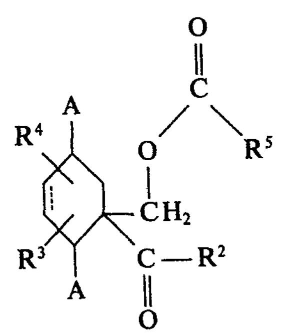

(IV)

dans laquelle A et $R^2$ à $R^4$ possèdent les significations définies pour la formule I et $R^5$ représente un atome d'halogène, de préférence de chlore, ou un reste —OAr, dans lequel Ar représente un groupe phényle éventuellement substitué, généralement dans un solvant et éventuellement en présence d'un catalyseur basique, à des températures de 0 à 12°C, avec une amine de la formule générale V

$$R^1{-}NH_2 \qquad (V)$$

dans laquelle $R^1$ possède les significations définies pour la formule I sans cependant pouvoir comporter une double ou une triple liaison en position $\alpha,\beta$ par rapport à l'atome d'azote, le composé

obtenu, s'il est non saturé, pouvant être soumis ensuite à une hydrogénation catalysée.

13. Composition pharmaceutique, contenant à côté de matières supports et de diluants usuels un composé de la formule(I) selon la revendication 1 comme substance active.

14. Composé de la formule (I) selon la revendication 1, utilisé comme substance hypnotique dans le traitement de troubles du sommeil ou comme substance sédative.